(19) Europäisches Patentamt
European Patent Office
Office européen des brevets

(11) EP 1 493 811 A1

(12) **EUROPEAN PATENT APPLICATION**
published in accordance with Art. 158(3) EPC

(43) Date of publication:
**05.01.2005 Bulletin 2005/01**

(21) Application number: **03715776.5**

(22) Date of filing: **08.04.2003**

(51) Int Cl.7: **C12N 15/11**, C12N 1/19, C12P 7/62 // C12N1:19, C12R1:72

(86) International application number:
**PCT/JP2003/004426**

(87) International publication number:
**WO 2003/085111 (16.10.2003 Gazette 2003/42)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **08.04.2002 JP 2002105240**

(71) Applicant: **KANEKA CORPORATION**
**Osaka-shi, Osaka 530-8288 (JP)**

(72) Inventors:
- **NAGAOKA, Tetsuya**
  **Kobe-shi, Hyogo 651-2124 (JP)**
- **YOKOMIZO, Satoru**
  **Akashi-shi, Hyogo 674-0092 (JP)**
- **MIYAMOTO, Kenji, 101, Nikkanhaimu Dai8**
  **Yokohama-shi, Kanagawa 223-0062 (JP)**
- **OSAKADA, Fumio**
  **Okayama-shi, Okayama 700-0063 (JP)**
- **MATSUMOTO, Keiji**
  **Nishinomiya-shi, Hyogo 663-8023 (JP)**
- **TAKAGI, Masamichi**
  **Fuchu-shi, Tokyo 183-0051 (JP)**
- **OTA, Akinori**
  **Saitama-shi, Saitama 331-0063 (JP)**

(74) Representative: **HOFFMANN - EITLE**
**Patent- und Rechtsanwälte**
**Arabellastrasse 4**
**81925 München (DE)**

(54) **NOVEL PROMOTERS**

(57) The present invention provides a production method of a copolymeric polyester which comprises culturing an ACT1 gene promoter shown under SEQ ID NO:9, a GAP3 gene promoter shown under SEQ ID NO: 10; a PMA1 gene promoter shown under SEQ ID NO: 11, and, a TEF1 gene promoter shown under SEQ ID NO:12; a plasmid which contains the gene expression unit comprising said promoter; a transformed cell as resulting from transformation of the said plasmid; and said transformed cell.

EP 1 493 811 A1

## Description

TECHNICAL FIELD

The present invention relates to a novel promoter enabling gene expression in yeasts belonging to the genus *Candida*. More particularly, it relates to a promoter enabling constitutive and highly efficient gene expression in yeast belonging to the genus *Candida* without dependence on the induction conditions such as culture conditions and medium conditions.

BACKGROUND ART

Advances in gene recombination technology have made it possible to produce useful proteins and useful chemicals, among others, using microorganisms. Gene Recombination systems in which *Escherichia coli* and *Bacillus subtilis*, which are prokaryotes, have been positively developed, and various useful substances have been produced using host-vector systems involving *Escherichia coli,* in particular. However, there are problems: proteins produced in *Escherichia coli* may form insoluble granules in cells and they cannot be undergone glycosylation, which is a characteristic feature of eukaryotes.

The development of systems in which yeasts, which are eukaryotes, are used as hosts has also advanced. Yeasts have been long utilized in brewing and baking and once have been produced for use in feedstuffs and, thus, their high safety has been guaranteed. Their ability to glycosylate proteins produced therein is one of the features distinguishing them from prokaryotes.

Host-vector systems have been developed not only in *Saccharomyces cerevisiae,* on which genetic findings are abundant, but also in the genera *Schwanniomyces, Kluyveromyces*, *Pichia, Hansenula, Yarrowia* and *Candida* (Klaus Wolf: Nonconventional Yeasts in Biotechnology, published by Springer).

Among those yeasts, some can grow utilizing straight hydrocarbon chains (n-alkanes) as the only carbon source. They are *Candida maltosa, Candida tropicalis* and the like, which belong to the genus *Candida, Hansenula polymorpha,* and *Yarrowia lipolytica*, among others. These yeasts have an enzyme system for oxidizing n-alkanes at the terminus thereof and thus can utilize them as energy sources by decomposing the long-chain carboxylic acids resulting from such oxidation to give acetyl-CoA, which serves as a substrate of the TCA cycle, by means of the peroxisome β oxidation system.

Such n-alkane-utilizing yeasts not only can grow on straight hydrocarbons as carbon sources but also are resistant to hydrophobic substances which inhibit the growth of general yeasts. Therefore, they are promising hosts for providing the field of reaction for producing useful substances by conversion of hydrophobic chemical substances. Thus, by constructing a gene expression system in such yeast, it becomes possible to construct a novel useful chemical substance production system in which no hazardous byproducts are produced and no energy is wasted.

Positive efforts have been made to construct gene expression systems in *Candida maltosa* among such n-alkane-utilizing yeasts. M. Kawamura et al. discovered a region causing high-efficiency transformation (transformation ability; hereinafter referred to as "TRA" for short) in *Candida maltosa* (M. Kawamura, et al., Gene, vol. 24, 157 (1983)). It was revealed that this region includes a sequence involved in autonomous replication in *Candida maltosa* (autonomously replicating sequence; hereinafter referred to as "ARS" for short) and a centromere sequence (hereinafter referred to as "CEN" for short). Until now, a low-copy-number vector having the whole of the TRA region and a high-copy-number vector deprived of the CEN region and expected to allow high-level expression of transgenes have been developed (M. Ohkuma, et al., Mol. Gen. Genet., vol. 249, 447 (1995)).

A promoter capable of functioning in *Candida maltosa* is required for gene expression in that yeast, and a plurality of promoters are now available therefor. *Candida maltosa* produces, in the presence of alkanes, high levels of enzymes involved in the n-alkane oxidation system. In particular, transcription of the genes encoding cytochrome P450 which can be participates in early-stage oxidation of alkanes (hereinafter such genes being referred to as "ALK" for short) are strongly induced (M. Ohokuma, et al., DNA and Cell Biology, vol. 14, 163 (1995)) and the transcription of genes encoding the β oxidation system are also induced (Y. Masuda, et al., Gene, vol. 167, 157 (1995)). The promoter of the ALK1 gene among the ALK gene group is most strongly induced by n-alkanes and can be utilized in gene expression. The promoter of the ALK2 or ALK5 gene is suited for use in the presence of fatty acids.

The promoter of the gene for phosphoglycerate kinase (hereinafter referred to as "PGK" for short) known as an enzyme involved in the glycolytic pathway is known to induce potent gene expression in the presence of glucose. The PGK promoter of *Candida maltosa* has been cloned by Y. Masuda et al. (Y. Masuda, et al., Curr. Genet., vol. 25, 412 (1994)). Furthermore, the GAL promoter having potent gene expression inducing activity in the presence of galactose has also been cloned (S. M. Park, et al., Yeast, vol. 13, 21 (1997)). The ALK promoter, PGK promoter and GAL promoter cloned as mentioned above can be utilized in gene expression in *Candida maltosa*.

In cases where such a carbon source as glucose is used, however, the ALK promoter will hardly function.

When fatty acids or n-alkanes are used as carbon sources, the PGK promoter will hardly function. Therefore, carbon sources suited for the production of a useful substance in *Candida maltosa* are not always adequate for potent gene expression in the production of that substance. Furthermore, the GAL promoter is induced only when galactose is used as the carbon source; hence, the GAL promoter may be said to be unsuited for commercial production since the use of galactose, which is expensive, is required.

Thus, a novel promoter capable of potent gene expression without any restriction as to the kind of carbon source has been desired for high-efficiency production of useful substances in *Candida maltosa*.

As regards *Saccharomyces cerevisiae*, on the other hand, promoter of the alcohol dehydrogenase 1 gene, glyceraldehyde-3-phosphate dehydrogenase 3 gene (hereinafter referred to as "GAP3" for short), PGK and GAL, among others, have already been cloned and have been widely utilized as promoters for the expression of foreign genes. It has already been revealed that the activity of these promoters is powerful when that yeast is used as the host. Thus, high expression levels can be expected even when *Candida maltosa* is used as the host. However, such promoter region genes have not been cloned as yet in *Candida maltosa*; therefore, the cloning thereof has been awaited.

Unlike *Saccharomyces cerevisiae*, however, no sexual generation is known of yeasts of the genus *Candida*. In many of them, the genome is diploid. Furthermore, it was reported that there is an abnormality in the reading of the genetic code. It is reported that the CUG codon generally encoding leucine is read as the codon for serine in *Candida cylindraceae* (Y. Kawaguchi, et al., Nature, vol. 341, 164 (1989)) or in *Candida maltosa* (H. Sugiyama, et al., Yeast, vol. 11, 43 (1995)). In this manner, yeasts of the genus Candida may be said to have properties markedly different from those of *Saccharomyces cerevisiae* and, therefore, it has remained unknown whether the *Saccharomyces cerevisiae*-derived promoter region as such can show comparative promoter activities in yeasts of the genus *Candida* as well.

SUMMARY OF THE INVENTION

In view of the above-mentioned state of the art, it is an object of the present invention to construct a promoter enabling potent gene expression in yeast, of the genus *Candida,* especially in *Candida maltosa* without being influenced by the kind of carbon source and thus provide a novel promoter enabling constitutive and high-efficiency production of useful substances.

As a result of intensive investigations made by them, the present inventors succeeded for the first time in identifying the actin synthetase 1 gene (hereinafter referred to as "ACT1" for short) promoter, glyceraldehydes-3-phosphate dehydrogenase 3 gene (hereinafter referred to as "GAP3" for short) promoter, protoplasmic membrane proton ATPase 1 gene (hereinafter referred to as "PMA1" for short) promoter and translational elongation factor 1 gene (hereinafter referred to as "TEF1" for short) promoter of *Candida maltosa*, and found that those promoters are at comparable or superior in promoter activity to the existing ALK promoter, PGK promoter and GAL promoter. Such findings have now led to completion of the present invention.

Thus, the present invention relates to an ACT1 gene promoter which comprises a DNA selected from among the following (a) to (d):

(a) a DNA shown under SEQ ID NO:9;
(b) a DNA containing the base sequence shown under SEQ ID NO:9 and having promoter activity;
(c) a DNA containing a base sequence derived from the base sequence shown under SEQ ID NO:9 by deletion, substitution or addition of at least one base and having promoter activity;
(d) a DNA derived from a yeast belonging to genus *Candida,* which hybridizes with base sequence of SEQ ID NO: 9 under stringent condition and has a promoter activity.

In addition, the present invention relates to a GAP3 gene promoter which comprises a DNA selected from among the following (a) to (d):

(a) a DNA shown under SEQ ID NO:10;
(b) a DNA containing the base sequence shown under SEQ ID NO:10 and having promoter activity;
(c) a DNA containing a base sequence derived from the base sequence shown under SEQ ID NO:10 by deletion, substitution or addition of at least one base and having promoter activity.
(d) a DNA derived from a yeast belonging to genus *Candida,* which hybridizes with base sequence of SEQ ID NO: 10 under stringent condition and has a promoter activity.

Furthermore, the present invention relates to a PMA1 gene promoter which comprises a DNA selected from among the following (a) to (d):

(a) a DNA shown under SEQ ID NO:11;
(b) a DNA containing the base sequence shown under SEQ ID NO:11 and having promoter activity;
(c) a DNA containing a base sequence derived from the base sequence shown under SEQ ID NO:11 by deletion, substitution or addition of at least one base and having promoter activity.
(d) a DNA derived from a yeast belonging to genus *Candida,* which hybridizes with base sequence of SEQ ID NO: 11 under stringent condition and has a promoter activity.

Moreover, the present invention relates to a TEF1 gene promoter
which comprises a DNA selected from among the following (a) to (d):

(a) a DNA shown under SEQ ID NO:12;
(b) a DNA containing the base sequence shown under SEQ ID NO:12 and having promoter activity;
(c) a DNA containing a base sequence derived from the base sequence shown under SEQ ID NO:12 by deletion, substitution or addition of at least one base and having promoter activity.
(d) a DNA derived from a yeast belonging to genus Candida, which hybridizes with base sequence of SEQ ID NO: 12 under stringent condition and has a promoter activity.

Furthermore, the present invention relates to a DNA
which comprises any one of the promoter mentioned above and a structural gene joined to the promoter sequence downstream therefrom.
Furthermore, the present invention relates to a gene expression unit
which comprises the DNA mentioned above and a terminator.
Furthermore, the present invention relates to a plasmid
which contains the gene expression unit mentioned above.
Furthermore, the present invention relates to a transformed cell as resulting from transformation of the DNA or the plasmid mentioned above into a host cell.
Furthermore, the present invention relates to a method of producing the copolymeric polyester resulting from copolymerization of 3-hydroxybutyric acid and 3-hydroxyhexanoic acid
which comprises culturing the transformed cell wherein the structural gene is an *Aeromonas caviae*-derived gene encoding an enzyme involved in the synthesis of the copolymeric polyester resulting from copolymerization of 3-hydroxybutyric acid and 3-hydroxyhexanoic acid.

DETAILED DESCRIPTION OF THE INVENTION

The ACT1 gene promoter, GAP3 gene promoter, PMA1 gene promoter and TEF1 gene promoter of *Candida maltosa* are novel promoters the sequences of which have been identified for the first time by the present inventors. In the following, they are described in detail.

(1) Cloning of novel promoters

The ACT1 gene encodes one of the enzymes involved in the synthesis of actin, which constitutes the cytoskeleton, the GAP3 gene for one of glycolytic pathway enzymes, the PMA1 gene for one of protoplasmic membrane proteins, which is said to account for about 10% of the protoplasmic membrane proteins in yeast of the genus *Saccharomyces*, and the TEF1 gene for one of translational elongation factors in protein synthesis, and all the corresponding promoters of *Saccharomyces cerevisiae* are known as potent promoters in that yeast.
For cloning the promoter regions of the ACT1 gene, GAP3 gene, PMA1 gene and TEF1 gene of *Candida maltosa* by hybridization, the present inventors first amplified, by PCR method, partial DNA sequences of the corresponding structural genes from *Saccharomyces cerevisiae* belonging to a relatively allied genus, and used them as probe fragments. For example, the sequence of the ACT1 gene of *Saccharomyces cerevisiae* has already been reported (Daniel, H. M. et al., Int. J. Syst. Evol. Microbiol., vol. 51, 1593 (2001)). Based on that sequence, PCR primers for amplifying the ACT1 gene of *Saccharomyces cerevisiae* can be synthesized. The sequence of the GAP3 structural gene of *Saccharomyces cerevisiae* has already been reported (Holland, M. J. et al., J. Biol. Chem., vol. 254, 5466 (1979)). Based on that sequence, PCR primers for amplifying the GAP3 gene of *Saccharomyces cerevisiae* can be synthesized. Further, the sequence of the PMA1 gene of *Saccharomyces cerevisiae* has already been reported by Capieaux, E. et al. (J. Biol. Chem., vol. 264, 7437 (1989)). Based on that sequence, PCR primers for amplifying the PMA1 structural gene of *Saccharomyces cerevisiae* can be synthesized. The sequence of the TEF1 structural gene of *Saccharomyces cerevisiae* has already been reported by Cottrelle, P., et al. (J. Biol. Chem., vol. 260, 3090 (1985)). Based on that sequence, PCR primers for amplifying the TEF1 structural gene of *Saccharomyces cerevisiae* can be synthesized.

The chromosomal DNAs of *Saccharomyces cerevisiae* and *Candida maltosa* can be isolated using commercial reagents, for instance. By using the thus-isolated chromosomal DNA of *Saccharomyces cerevisiae* as a template for PCR and carrying out PCR using the DNA primers synthesized as mentioned above, it is possible to amplify a part each of the ACT1, GAP3, PMA1 and TEF1 structural genes corresponding to each respective primer combination. Hybridization detection probes can be prepared by labeling the thus-amplified DNA fragments with a radioactive compound or alkaline phosphatase, for instance.

The lengths of fragments presumably containing the respective desired promoter sequences can be estimated by isolating the *Candida maltosa* chromosomal DNA, fragmenting it with an appropriate restriction enzyme to subject to agarose gel electrophoresis, and carrying out Southern hybridization using thus-obtained DNA with'the above-mentioned detection probes. For each promoter, a gene library can be constructed by cleaving the *Candida maltosa* chromosome with the restriction enzyme used for the above Southern hybridization, and using a cloning vector. This library is transformed so that an appropriate number of colonies may appear on an antibiotic-containing agar medium and then cultured on a nitrocellulose membrane, and this membrane is subjected to alkaline denaturation, neutralization, washing and drying and then to hybridization, whereby a chromosomal DNA fragment containing each desired promoter of *Candida maltosa* can be cloned. Each DNA fragment obtained in this manner contains not only the respective promoter region but also the respective structural gene. Therefore, in view of their similarity (homology) to those corresponding known promoters or structural genes whose base sequences are known, such as *Saccharomyces cerevisiae*, the promoter regions can be identified and distinguished from the structural genes using general techniques to determine the base sequences of the promoters.

In the manner mentioned above, the novel ACT1 gene promoter, GAP3 gene promoter, PMA1 gene promoter and TEF1 gene promoter of the invention can be obtained. The base sequences of these promoters are shown under SEQ ID NOs:9, 10, 11 and 12, respectively.

The above-mentioned promoters of the present invention may be obtained by using the techniques mentioned above. Since the base sequences thereof have been identified, it is also possible to obtained them by chemical synthesis.

The promoters of the present invention may be not only the above DNAs shown under SEQ ID numbers: 9, 10, 11 and 12 but also DNAs comprising a DNA containing any of the sequences mentioned above or a DNA derived from any of the sequences mentioned above by deletion, substitution or addition of at least one base. Furthermore, it may be a DNA derived from a yeast belonging to genus *Candida*, which hybridizes with the base sequence of above SEQ ID numbers under stringent condition and has a promoter activity.

The "DNA comprising a DNA containing any of the sequences mentioned above" or "DNA derived from any of the sequences mentioned above by deletion, substitution or addition of at least one base" means any DNA derived by the deletion, addition, insertion and/or substitution of a number of bases of the order which can be deleted, added, inserted and/or substituted by the methods well known in the art, for example as described in, inter alia, Protein, Nucleic Acid, Enzyme, Supplemental Issue: Gene Amplification PCR Technology TAKKAJ 35 (17), 2951-3178 (1990) or Henry A. Erlich (ed.), PCR Technology (the translation edited by Ikunoshin Kato) (1990).

The "DNA derived from a yeast belonging to genus Candida, which hybridizes with the base sequence of above SEQ ID numbers under stringent condition" means a DNA obtained by colony hybridization, plaque hybridization, Southern hybridization or the like hybridization technique.

The DNA of the present invention preferably comprises a base sequence showing a homology of not less than 80% to the base sequence shown under above SEQ ID number. The homology is preferably not less than 90%, more preferably not less than 95%, still more preferably not less than 98%.

The "homology" is calculated by aligning two nucleotide sequences to be compared in the optimum format, counting the matched base (A, T, C, G, U or I) positions between the two sequences, dividing the count by the total number of bases to be compared, and multiplying the product by 100. Specifically, this calculation can be made using an analytical software such as Hitachi Soft Engineering's DNASIS, Software Development's GENETYX, or Finland CSC's Clustal X, for instance.

These DNAs may be easily obtained by the deletion, addition, insertion and/or substitution, using the above well-known methods in the art, of at least one base to the DNA shown under above SEQ ID numbers, or by carrying out the hybridization according to the methods described in Molecular Cloning, 2nd Edition (Cold Spring Harbor Laboratory Press, 1989).

In the present invention, the promoter "having a promoter activity" means the promoter whose enzyme activity, measured by the function analysis mentioned hereinafter, is not less than 50% relative to that of the DNA of the above SEQ ID number measured under the same condition. The enzyme activity of the promoter is preferably not less than 70%, more preferably not less than 80%, particularly preferably not less than 90%.

(2) Analysis of promoter functions

The functions of the novel promoters can be analyzed by quantitating the mRNAs transcribed as a result of functioning of the promoters or the gene products translated from those mRNAs.

When a structural gene is joined to the promoters of the invention downstream therefrom, the promoters can allow the structural gene to perform its function. The structural gene which can be used in the invention is not particularly restricted, but includes a gene encoding a enzyme involved in the synthesis of polyhydroxyalkanoate (particularly, copolymeric polyester P(3HB-co-3HH) resulting from copolymerization of 3-hydroxybutyric acid and 3-hydroxyhexanoic acid, a antibody gene, a gene encoding a useful protein such as lymphokine, or the like genes.

Furthermore, if necessary, the promoter of the invention and a structural gene joined downstream therefrom, together with a terminator, can be used as gene expression units. The terminator to be used here may be any appropriate one among the known terminators provided that it can be used in the objective expression system. Said terminator is not particularly restricted, but there may be mentioned ALK1 or GCN4, among others.

It is also possible to insert such gene expression units in a plasmid for utilization of the units. The plasmid which may be used in the present invention is not particularly restricted, but includes pUTU1 or pBTH10B (Nakazawa, et al., J.Bacteriol., vol. 179, 5030(1997)), among others.

In accordance with the present invention, it is possible to transform the plasmid referred to above into host cells constituting an expression system for preparing transformed cells and then culture these transformed cells to cause expression of the structural gene transformed. In place of plasmid transformation, a DNA comprising any of the promoters of the invention and a structural gene joined downstream therefrom may be incorporated directly into the host chromosome. The host to be used here is not particularly restricted provided that at least one promoter of the invention can function therein, but a yeast belonging to the genus *Candida* is preferred, and *Candida maltosa* is particularly preferred.

The host cell can be transformed with the plasmid of the present invention in the conventional manner. For example, the calcium method (E. M. Lederberg, et al., J. Bacteriol., vol. 119, 1072 (1974)) or the electroporation method (Current Protocols in Molecular Biology, vol. 1, para. 1.8.4, 1994) can be used. Commercial transformation kits such as Fast Track™-Yeast Transformation Kit SM (Geno Technology) may also be utilized.

The function analysis of the novel promoters using the transformants obtained can be carried out by culturing them using a carbon source (s) utilizable by the same transformants. For the culture of said transformant, a medium generally used in the culture of a transformant may be used. Such medium may be prepared by adding a carbon source to YPD medium (enzyme extract 1%, polypepton 2%), YNB medium (Yeast nitrogen base 0.67%) or the like. In addition, a synthesized medium may be also used. The carbon source which may be used in the invention is not particularly restricted, but includes sugars such as glucose or maltose; n-alkanes, fatty acids, and oils and fats, among others. The temperature at the culture and culturing time are not particularly restricted as long as the condition is suitable for a transformant to growth. Preferably, said culture may be carried out at 10 to 40°C for 2 through 72 hours. The function analysis of promoter of the cultured cell can be carried out by analyzing culture medium or proteins in the cell. There is no need to fracture the cell when an enzyme, which is expressed from a gene joined downstream from a promoter, or the like are expressed outside the cell. On the other hand, when the enzyme stays in the cell, it is necessary to treat it. The method for treating a cell is not particularly restricted. But, among them, there may be mentioned a method in which the cell is subjected to digestion by cell wall lytic enzyme, such as Zymolyase, and then to sonication, freeze-thawing, or the like treatment. Furthermore, direct physical fracturing with glass bead may be also used.

Function analysis of the promoter may be carried out by the method which can detect specifically an activity or a content of, such as an enzyme, which is expressed from a gene joined downstream from a promoter. There is no limitation for the analysis method, but the analysis can be carried out, for example, by constructing expression vectors for the expression of the *Aeromonas caviae*-derived enzyme (hereinafter referred to as "ORF2S") involved in the synthesis of a copolymer of 3-hydroxybutyric acid and 3-hydroxyhexanoic acid, namely copolyester P (3HB-co-3HH), using the respective promoter regions cloned as described above under (1), and then determining the activity of that enzyme by the method described in the literature (Valentin, H. E., et al., Appl. Microbiol. Biotechnol., vol. 40, 699 (1994)). Particularly, the enzyme activity may be measured by a simple and easy manner in the case of a measurement of a promoter using ORF2S, by subjecting the CoA-SH which becomes free on incorporation of (R)-3-hydroxybutylyl-CoA by ORF2S, to reaction with DTNB (5,5'-dithiobis(2-nitrobenzoic acid)), which can be reacted with CoA-SH in equimolar ratio, and, at the same time, measuring increase per unit time of absorption at about 412 nm derived from ionized TNB (2- nitro-5-mercaptobenzoic acid) generated by the reaction with free CoA-SH.

By normalization using protein content in the fractured cell-containing solution, the value of enzyme activity obtained can be indicated as an enzyme specific activity expressed by the promoter of the invention; thus, it will be possible to compare a promoter activity of one another.

Among the promoters of the present invention, the ACT1 gene promoter, PMA1 gene promoter and TEF1 gene promoter are all promoters for the genes essential for growth and, therefore, can function under conditions under which Candida maltosa can grow, without being restricted by the carbon source species. The GAP3 gene promoter is the promoter for an enzyme involved in the glycolytic pathway and, from the viewpoint that very strong expression can be expected even when glucose is used as the carbon source, it has a property that any known promoter cannot have.

According to the production method of the present invention, said copolymeric polyester may be produced by culturing said transformed cell, the structural gene of which is the gene derived from *Aeromonas caviae*, encoding a enzyme involved in the synthesis of copolymeric polyester resulting from copolymerization of 3-hydroxybutyric acid and 3-hydroxyhexanoic acid.

As a carbon source which can be used in said culture, any one that can be utilized by the transformant may be used. In addition, culture medium containing other nutrition sources than carbon, such as nitrogen source, inorganic salts, or other culture medium containing an organic nutrition source, may be also used. Any temperature condition, in which the cell can grow, may be applied, but preferably it may be between 20 and 40°C. Culturing time is not particularly restricted, but it may be 1 to 7 days. Then, polyester may be recovered from the cultured cell or medium resulting from the culture.

As the carbon source, carbohydrate such as glucose, glycerin and sucrose, oils and fats, fatty acids, further n-paraffin and the like may be used. As the fats and oils, for instance, there may be mentioned rapeseed oil, coconut oil, palm oil, palm kernel oil, among others. As the fatty acids, for instance, a saturated/unsaturated fatty acid such as hexanoic acid, octanoic acid, decanoic acid, lauric acid, oleic acid, palmitic acid, linoleic acid, linolenic acid, myristic acid, or a fatty acid derivative such as an ester or a salt of these fatty acid, may be mentioned. As one example, culture of *Candida maltosa* may be carried out with oils and fats as a carbon source. In the case of a transformant which cannot utilize, or cannot effectively utilize, lipase may be added to the medium to improve the utilization efficiency. Furthermore, oils and fats utilizing-ability can be attached by transforming lipase gene.

As the nitrogen source, for instance, ammonia, ammonium salt such as ammonium chloride, ammonium sulfate and ammonium phosphate; peptone, meat extract, yeast extract and the like may be mentioned. As inorganic salts, for instance, Dipotassium Hydrogenphosphate, Potassium Dihydrogenphosphate, magnesium phosphate, magnesium sulfate, sodium chloride and the like are included.

As the other organic nutrition sources, for instance, amino acids (i.e. glycine, alanine, serine, threonine, proline, etc.), vitamins (i.e. vitamin B1, vitamin B12, biotin, nicotinic-acid amide, pantothenic acid, vitamin C, etc.), and the like may be mentioned.

In the present invention, recovery of polyester from the cell may be carried out by the following method. After completion of the culture, the cell is separated from culture medium by centrifugal separator and the like, then the cell is washed with distilled water, methanol or the like, and dried. Polyester is extracted from the cell with an organic solvent such as chloroform. Cell constituent is removed from the organic solvent solution containing the polyester by, for example, filtration. A poor solvent such as methanol and hexane is added to thus-obtained filtrate to precipitate polyester. Supernatant is removed by filtration or centrifugation, and thus-obtained, precipitated polyester is dried to recover polyester.

BRIEF DESCRIPTION OF THE DRAWINGS

Fig. 1 represents a restriction map of a plasmid named pUAL-ORF2S, which is used for the plasmid construction according to Example 9.

Fig. 2 represents a restriction map of a plasmid named pSTV-ALK1-ORF2S, which is used for the plasmid construction according to Examples 9 to 13.

Fig. 3 represents a restriction map of a plasmid named pUTA1, which is used for the plasmid construction according to Examples 9 to 13.

Fig. 4 represents a restriction map of a plasmid named pUTA-AZK1-ORF2S constructed in Example 9.

Fig. 5 represents a restriction map of a plasmid named pUTA-ACT1-ORF2S, which is one aspect of the invention, constructed in Example 10.

Fig. 6 represents a restriction map of a plasmid named pUTA-GAP3-ORF2S, which is one aspect of the invention, constructed in Example 11.

Fig. 7 represents a restriction map of a plasmid named pUTA-PMA1-ORF2S, which is one aspect of the invention, constructed in Example 12.

Fig. 8 is a schematic representation of a plasmid named pUTA-TEF1-ORF2S, which is one aspect of the invention, constructed in Example 13.

BEST MODE FOR CARRING OUT THE INVENTION

The following examples illustrate the present invention more specifically. However, these examples are by no means limitative of the scope of the invention.

(Example 1) Preparation of yeast chromosomal DNAs

The chromosomal DNAs of *Saccharomyces cerevisiae* and *Candida maltosa* were prepared using E.Z.N.A. Yeast DNA Kits (products of OMEGA BIOTEK). The preparation procedure was as described in the protocol attached to the kits.

(Example 2) Amplification of fragments of the ACT1, GAP3, PMA1 and TEF1 genes of *Saccharomyces cerevisiae*

Fragments of the *Saccharomyces cerevisiae* ACT1, GAP3, PMA1 and TEF1 genes the base sequences of which are already known were amplified as follows. The fragments were amplified by PCR using synthetic DNAs specified under SEQ ID NOs:1 and 2 (for ACT1), SEQ ID NOs:3 and 4 (for GAP3), SEQ ID NOs:5 and 6 (for PMA1) and SEQ ID NOs:7 and 8 (for TEF1) as primers and the *Saccharomyces cerevisiae* chromosomal DNA prepared in Example 1 as a template together with TaKaRa Ex Taq polymerase (product of Takara Shuzo). The conditions employed were as described in the protocol attached to the kit. More specifically, water was added, to make 0.1 ml, to a mixture of 1 μg of the template DNA, two primers each in an amount to give a final concentration of 1 μM, 2.5 U of Ex Taq polymerase, 0.01 ml of the attached buffer and 0.008 ml of the attached dNTP mixture. This was subjected to 25 cycles of PCR (each cycle comprising: 15 seconds at 98°C, 1 minute at 55°C, and 1 minute at 72°C) to thereby amplify an ACT1 gene fragment of about 400 bp, or a GAP3 gene fragment of about 1 kb, or a PMA1 gene fragment of about 2.8 kb, or a TEF1 gene fragment of about 1.5 kb, of *Saccharomyces cerevisiae*.

(Example 3) Preparation of labeled probe fragments, hybridization, washing, and detection of positive clones

The respective probe fragments amplified in the above manner were labeled with alkaline phosphatase using Amersham-Pharmacia's Gene images AlkPhos kit according to the protocol attached thereto.
The hybridization was carried out overnight at 55°C using Amersham-Pharmacia's Gene images AlkPhos kit according to the protocol attached thereto.
The washing was carried out at 55°C and at room temperature using Amersham-Pharmacia's Gene images AlkPhos kit according to the protocol attached thereto.
Positive clones were detected using Amersham-Pharmacia' s CDP-Star kit according to the protocol attached thereto.

(Example 4) Cloning of the *Candida maltosa* ACT1 gene promoter region

The chromosomal DNA of Candida maltosa as prepared in Example 1 was cleaved with the restriction enzyme *Bgl*II and fragments of about 1 kb to 3 kb were extracted from the gel by following agarose gel electrophoresis. These fragments were joined to pUC19, which was treated with the restriction enzyme *Bam*HI, and the resulting recombined plasmids were used to transform the *Escherichia coli* DH5α strain. About 3,000 transformed colonies were screened to hybridization with the alkaline phosphatase-labeled *Saccharomyces cerevisiae* ACT1 gene fragment as a probe. As a result, 6 colonies of positive clone were obtained and, upon partial base sequence determination of the inserted fragment, the insert was found to be a gene containing the ACT1 promoter region of *Candida maltosa*. The partial sequence of the cloned fragment is shown under SEQ ID NO:9.

(Example 5) Cloning of the *Candida maltosa* GAP3 gene promoter region

The chromosomal DNA of *Candida maltosa* as prepared in Example 1 was cleaved with the restriction enzyme *Eco*RI and fragments of about 7 kb to 9 kb were extracted from the gel by following agarose gel electrophoresis. These fragments were joined to pUC19, which was treated with the same restriction enzyme, and the resulting recombined plasmids were used to transform the *E. coli* DH5α strain. About 2,000 transformed colonies were screened to hybridization with the alkaline phosphatase-labeled *Saccharomyces cerevisiae* GAP3 gene fragment as a probe. As a result, 2 colonies of positive clone were obtained and, upon partial base sequence determination of the inserted fragment, the insert was found to be a gene containing the GAP3 promoter region of *Candida maltosa*. The partial sequence of the cloned fragment is shown under SEQ ID NO:10.

(Example 6) Cloning of the *Candida maltosa* PMA1 gene promoter region

The chromosomal DNA of *Candida maltosa* as prepared in Example 1 was cleaved with the restriction enzyme *Xba*I and fragments of about 2 kb to 4 kb were extracted from the gel by following agarose gel electrophoresis. These fragments were joined to pUC19, which was treated with the same restriction enzyme, and the resulting recombined plasmids were used to transform the *E. coli* strain DH5α. About 5,000 transformed colonies were screened to hybridization with the alkaline phosphatase-labeled *Saccharomyces cerevisiae* PMA1 gene fragment as a probe. As a result, 5 colonies of positive clone were obtained and, upon base sequence determination of the inserted fragment, the insert was found to be a gene containing the PMA1 promoter region of *Candida maltosa*, as shown under SEQ ID NO:11.

(Example 7) Cloning of the *Candida maltosa* TEF1 gene promoter region

The chromosomal DNA of *Candida maltosa* as prepared in Example 1 was cleaved with the restriction enzymes EcoRI and PstI and fragments of about 2 kb to 4 kb were extracted from the gel by following agarose gel electrophoresis. These fragments were joined to pUC19 treated with the same restriction enzymes, and the resulting recombined plasmids were used to transform the *E. coli* DH5α strain. About 400 transformed colonies were screened to hybridization with the alkaline phosphatase-labeled *Saccharomyces cerevisiae* TEF1 gene fragment as a probe. As a result, 7 colonies of positive clone were obtained and, upon base sequence determination of the inserted fragment, the insert was found to be a gene containing the TEF1 promoter region of *Candida maltosa*, as shown under SEQ ID NO:12.

(Example 8) Synthesis of a gene involved in polyester synthesis

An enzyme gene involved in polyester synthesis was synthesized based on the amino acid sequence of the Aeromonas *caviae*-derived polyhydroxyalkanoate (PHA) synthetase (T. Fukui, et al., FEMS Microbiology Letters, vol. 170, 69 (1999)). Since *Candida maltosa* is an yeast, which translates the CTG codon into serine, not into leucine, CTG was not assigned to the leucine codon. Those codons, which are frequently used in *Candida maltosa*, were preferentially selected as the codons corresponding to the respective amino acids. As for the frequency of the codon, the monograph "Nonconventional Yeasts in Biotechnology" written by Klaus Wolf (published by Springer) was consulted. The PHA-synthesizing enzyme gene (hereinafter referred to as "ORF2S" for short; SEQ ID NO:13) was thus designed and the ORF2S gene portion was totally synthesized.

(Example 9) Construction of an ORF2S expression vector using the *Candida maltosa* ALK1 promoter

For the expression of the above ORF2S in *Candida maltosa*, the promoter ALK1p (SEQ ID NO:15) of the *Candida maltosa* Alk1 gene was joined to the 5' upstream thereof, and the terminator ALK1t (SEQ ID NO:14) of the *Candida maltosa* Alk1 gene to the 3' downstream thereof. The restriction enzyme sites for joining the promoter and terminator to the structural gene were produced by utilizing the PCR method. For the promoter portion, PCR was carried out using the DNAs shown under SEQ ID NO:16 and SEQ ID NO:17 with the promoter specified under SEQ ID NO:15 as the template, and an ALK1p fragment having a *Pvu*II site at the 5' terminus and an *Eco*RI site at the 3' terminus was prepared. For the terminator portion, PCR was carried out using the DNAs shown under SEQ ID NO:18 and SEQ ID NO:19 with the terminator specified under SEQ ID NO:14 as the template, and an ALK1t fragment having a *Hind*III site at the 5' terminus and an *Eco*RV site at the 3' terminus was prepared. The ALK1p fragment was joined to pUCNT (described in WO 94/03613) at the *Pvu*II-*Eco*RI site thereof, and the ALK1t fragment was joined to pUCNT at the HindIII-SspI site thereof, whereby pUAL1 was constructed. Then, the ORF2S fragment was joined to pUAL1 at the *Nde*I-*Pst*I site thereof,' whereby the plasmid pUAL-ORF2S (Fig. 1) was constructed. Then, this plasmid was partially cleaved with *Sal*I, and the *Sal*I site was converted to an XhoI site using an XhoI linker (product of Takara Shuzo). The thus-modified plasmid was once cleaved with *Pvu*I and *Pvu*II and joined to the *Pvu*I and *Sma*I fragment of pSTV28 (product of Takara Shuzo), whereby pSTV-ALK1ORF2S shown in Fig. 2 was constructed.

Furthermore, pUTA1 (Fig. 3), a vector, the marker gene of which was changed from uracil to adenine by using pUTU1 (M. Ohkuma, et al., J. Biol. Chem., vol. 273, 3948 (1998)) and the *Candida maltosa* ADE1 gene (Genebank D00855), was used. It was constructed by eliminating the URA3 gene from pUTU1 using *Xho*I and joining the ADE1 gene excised using *Sal*I to the remainder.

A fragment containing the promoter, ORF2S and terminator was prepared from pSTV-ALK1-ORF2S using *Eco*T22I and inserted into pUTA1 at the *Pst*I site thereof, whereby pUTA-ALK1-ORF2S shown in Fig. 4 was constructed.

(Example 10) Construction of an ORF2S expression vector using the *Candida maltosa* ACT1 promoter

An ORF2S expression vector was constructed using the Candida maltosa ACT1 promoter region cloned in Example 4, as follows. A fragment having a restriction enzyme *Eco*T22I site at the 5'-side terminus and a restriction enzyme *Nde*I site at the 3' -side terminus was obtained by carrying out PCR using the synthetic DNAs shown under SEQ ID NOs:20 and 21 as the primers and the *Candida maltosa* ACT1 promoter region-containing fragment (SEQ ID NO:9) as the template, and this fragment was treated with *Eco*T22I and *Nde*I. pSTV-ALK1-ORF2S was treated in the same manner with *Eco*T22I and *Nde*I to prepare a fragment free of the ALK promoter. The two fragments were joined together to construct pSTV-ACT1-ORF2S. By treating this plasmid with the restriction enzyme *Eco*T22I, ACT1-ORF2S fragment was prepared. This fragment was inserted into the PstI site of the pUTA1 obtained in Example 9, whereby an expression vector, pUTA-ACT1-ORF2S, was constructed as shown in Fig. 5.

(Example 11) Construction of an ORF2S expression vector using the *Candida maltosa* GAP3 promoter

An ORF2S expression vector was constructed using the *Candida maltosa* GAP3 promoter region cloned in Example 5, as follows. A fragment having a restriction enzyme *Xho*I site at the 5'-side terminus and a restriction enzyme NdeI site at the 3'-side terminus was obtained by carrying out PCR using the synthetic DNAs shown under SEQ ID NOs:22 and 23 as the primers and the *Candida maltosa* GAP3 promoter region-containing fragment (SEQ ID NO:10) as the template, and this fragment was treated with *Xho*I and *Nde*I. The pSTV-ALK1-ORF2S plasmid was treated in the same manner with *Xho*I and *Nde*I to prepare a fragment free of the ALK promoter. The two fragments obtained were joined together to construct pSTV-GAP3-ORF2S. This plasmid was treated with the restriction enzymes *Xho*I and *Sal*I to prepare a GAP3-ORF2S fragment. The pUTA1 plasmid was treated with *Sal*I. The two fragments thus obtained were joined together, whereby an expression vector, pUTA-GAP3-ORF2S, was constructed as shown in Fig. 6.

(Example 12) Construction of an ORF2S expression vector using the *Candida maltosa* PMA1 promoter

An ORF2S expression vector was constructed using the *Candida maltosa* PMA1 promoter region cloned in Example 6, as follows. A fragment having a restriction enzyme *Xho*I site at the 5'-side terminus and a restriction enzyme NdeI site at the 3'-side terminus was obtained by carrying out PCR using the synthetic DNAs shown under SEQ ID NOs:24 and 25 as primers and the *Candida maltosa* PMA1 promoter region-containing fragment (SEQ ID NO:11) as the template, and this fragment was treated with *Xho*I and *Nde*I. The pSTV-ALK1-ORF2S plasmid was treated in the same manner with *Xho*I and *Nde*I to prepare a fragment free of the ALK promoter. The two fragments obtained were joined together to construct pSTV-PMA1-ORF2S. This plasmid was treated with the restriction enzymes XhoI and SalI to prepare a PMA1-ORF2S fragment. The pUTA1 plasmid obtained in Example 9 was treated with *Sal*I. Both the fragments obtained were joined together, whereby an expression vector, pUTA-PMA1-ORF2S, was constructed as shown in Fig. 7.

(Example 13) Construction of an ORF2S expression vector using the *Candida maltosa* TEF1 promoter

An ORF2S expression vector was constructed using the *Candida maltosa* TEF1 promoter region cloned in Example 7, as follows. A fragment having a restriction enzyme *Xho*I site at the 5'-side terminus and a restriction enzyme *Nde*I site at the 3'-side terminus was obtained by carrying out PCR using the synthetic DNAs shown under SEQ ID NOs:26 and 27 as the primers and the Candida maltosa TEF1 promoter region-containing fragment (SEQ ID NO:12) as the template, and this fragment was treated with *Xho*I and *Nde*I. The pSTV-ALK1-ORF2S plasmid obtained in Example 9 was treated in the same manner with *Xho*I and *Nde*I to prepare a fragment free of the ALK promoter. The two fragments obtained were joined together to construct pSTV-TEF1-ORF2S. This plasmid was treated with the restriction enzymes *Xho*I and *Sal*I to prepare a TEF1-ORF2S fragment. The pUTA1 plasmid obtained in Example 9 was treated with *Sal*I. The two fragments thus obtained were joined together, whereby an expression vector, pUTA-TEF1-ORF2S, was constructed as shown in Fig. 8.

(Example 14) Isolation of a *Candida maltosa* transformed colonies

The *Candida maltosa* AC16 strain (Deposit based on Budapest Treaty, International depository authority: National Institute of Advanced Industrial Science and Technology, International Patent Organism Depositary (1-1-3 Higashi, Tsukuba, Ibaraki, Japan), deposited on November 15, 2000, accession No. FERM BP-7366) was transformed by the electroporation method. The organism was precultured overnight on YPD medium at 30°C. One milliliter of the culture fluid was inoculated into 100 ml of the same medium placed in a 500-ml Sakaguchi flask and cultured at 30°C for about 7 hours. After 10 minutes of centrifugation at 3,000 rpm and at room temperature, cells were washed with three portions (each about 50 ml) of an ice-cooled 1 M sorbitol solution. The cells were suspended in 3 ml of the same solution, and the suspension was divided into 0.1-ml portions and stored at -80°C until use as cells to be transformed. For transformation, ECM 600M (product of BTX) was used. Specifically, 0.1 ml of the cells to be transformed and about 1 μg of any one of the expression vector DNAs constructed in Examples 9 to 13 were placed in a cuvette having a gap width of 2 mm, electric pulses were applied thereto under the condition: mode 2.5 kV, voltage 1. 9 kV, and resistance 246Ω. Immediately thereafter, the cuvette was ice-cooled, 0.5 ml of 1 M sorbitol was added, and the mixture was maintained at room temperature for 1 hour and then cultured on a YNB selection plate at 30°C. The selection plate used was composed of 0.67 w/v % Yeast Nitrogen Base without amino acid (product of Difco), 2 w/v % glucose, and 2 w/v % Bacto Agar (product of Difco).

By the above operations, 5 kinds of transformants, each containing any one of the expression vector constructed in Example 9 to 13, were obtained.

(Example 15) Promoter function analysis

Each transformant obtained in Example 14 was precultured overnight on 0.67 w/v % Yeast Nitrogen Base without amino acid (product of Difco) plus 2 w/v % glucose. Then, 2.5 ml of the preculture was inoculated into 50 ml of the same medium placed in a 500-ml Sakaguchi flask and cultured at 30°C for 24 hours. Only in a comparative example, namely in a culture of an ALK1 promoter-containing expression vector, 2 w/v % of n-dodecane was used as the carbon source. A portion, corresponding to about 10 ml, of the culture fluid was centrifuged at 3,000 rpm at room temperature for 10 minutes, and cells were washed with physiological saline and again centrifuged under the same conditions. The cell bodies were suspended in 1 ml of a 0.5 M potassium phosphate solution (pH 7.2), the suspension was mixed with the same volume of glass beads for fracturing yeast cells (0.45 mm; product of Biospec Products), and the cells were fractured by five times (each for 1 minute) of treatment on Mini Bead Beater (product of Biospec Products), followed by 10 seconds of centrifugation at 3,000 g on a centrifuge. The supernatant was recovered and used as an ORF2S activity assay sample. With the sample, the protein concentration was measured using Protein Assay Kit (product of BioRad) and the enzyme activity was determined as described in Valentin, H. E., et al., Appl. Microbiol. Biotechnol., vol. 40, 699 (1994).

Specifically, 0.01213 ml of aqueous solution containing 0.01 ml of cell fractured fluid and 7 mg/ml of (R)-3-hydroxybutylyl-CoA (product of Sigma), and 3.8 mg/ml of DTNB (product of Sigma) dissolved in 0.5 M potassium phosphate solution (pH 7.2) were added into 0.338 ml of water, and mixed. Then, absorbance variance at 412 nm of thus-obtained mixture was measured at room temperature, for 5 minutes. The measurement of the absorbance was carried out using a spectrophotometer produced by Shimadzu. The activity was calculated by the following formula.

$$\text{Activity (U/ml)} = \Delta A_{412}/\text{min} \times 10^3 \times V_T / \varepsilon_{412} \times V_E$$

In the formula, $\Delta A_{412}$/min represents an increase of absorbance per minute, $V_T$ represents an amount of reaction mixture, $\varepsilon_{412}$ is 15.6 X $10^3$ ($m^{-1} \cdot cm^{-1}$), and $V_E$ represents an amount of an enzyme fluid.

The enzyme activity is expressed in terms of specific activity (U/mg) as in the above-cited document and the following formula.

$$\text{Specific activity (U/mg)} = \text{Activity(U/ml)} / \text{Protein Concentration (mg/ml)}$$

The results thus obtained are shown in Table 1. From these results, it was revealed that the ACT1, GAP3, PMA1 and TEF1 promoter regions cloned in accordance with the present invention are promoters capable of functioning in *Candida maltosa* cells and are at least comparable in efficiency of the function to the ALK promoter.

Table 1

| Promoter | Enzyme activity (U/mg) |
|---|---|
| ACT1 | 0.021 |
| GAP3 | 0.018 |
| PMA1 | 0.020 |

Table 1 (continued)

| Promoter | Enzyme activity (U/mg) |
|---|---|
| TEF1 | 0.025 |
| ALK1 | ' 0.021 |

INDUSTRIAL APPLICABILITY

Thus, the present invention has made it possible to carry out highly efficient expression of useful genes in yeasts of the genus *Candida,* in particular in *Candida maltosa.*

**Claims**

1. An ACT1 gene promoter which comprises a DNA selected from among the following (a) to (d):

(a) a DNA shown under SEQ ID NO:9;
(b) a DNA containing the base sequence shown under SEQ ID NO:9 and having promoter activity;
(c) a DNA containing a base sequence derived from the base sequence shown under SEQ ID NO:9 by deletion, substitution or addition of at least one base and having promoter activity;
(d) a DNA derived from a yeast belonging to genus Candida, which hybridizes with base sequence of SEQ ID NO:9 under stringent condition and has a promoter activity.

2. A DNA which comprises the ACT1 gene promoter according to Claim 1 and a structural gene joined to the promoter sequence downstream therefrom.

3. A gene expression unit which comprises the DNA according to Claim 2 and a terminator.

4. A plasmid which contains the gene expression unit according to Claim 3.

5. The plasmid according to Claim 4, which is pUTA-ACT1-ORF2S.

6. A GAP3 gene promoter which comprises a DNA selected from among the following (a) to (d):

(a) a DNA shown under SEQ ID NO:10;
(b) a DNA containing the base sequence shown under SEQ ID NO:10 and having promoter activity;
(c) a DNA containing a base sequence derived from the base sequence shown under SEQ ID NO:10 by deletion, substitution or addition of at least one base and having promoter activity.
(d) a DNA derived from a yeast belonging to genus *Candida,* which hybridizes with base sequence of SEQ ID NO:10 under stringent condition and has a promoter activity.

7. A DNA which comprises the GAP3 gene promoter according to Claim 6 and a structural gene joined to the promoter sequence downstream therefrom.

**8.** A gene expression unit
which comprises the DNA according to Claim 7 and a terminator.

**9.** A plasmid
which contains the gene expression unit according to Claim 8.

**10.** The plasmid according to Claim 9,
which is pUTA-GAP3-ORF2S.

**11.** A PMA1 gene promoter
which comprises a DNA selected from among the following (a) to (d):

(a) a DNA shown under SEQ ID NO:11;
(b) a DNA containing the base sequence shown under SEQ ID NO:11 and having promoter activity;
(c) a DNA containing a base sequence derived from the base sequence shown under SEQ ID NO:11 by deletion, substitution or addition of at least one base and having promoter activity.
(d) a DNA derived from a yeast belonging to genus *Candida,* which hybridizes with base sequence of SEQ ID NO:11 under stringent condition and has a promoter activity.

**12.** A DNA
which comprises the PMA1 gene promoter according to Claim 11 and a structural gene joined to the promoter sequence downstream therefrom.

**13.** A gene expression unit
which comprises the DNA according to Claim 12 and a terminator.

**14.** A plasmid
which contains the gene expression unit according to Claim 13.

**15.** The plasmid according to Claim 14,
which is pUTA-PMA1-ORF2S.

**16.** A TEF1 gene promoter
which comprises a DNA selected from among the following (a) to (d):

(a) a DNA shown under SEQ ID NO:12;
(b) a DNA containing the base sequence shown under SEQ ID NO:12 and having promoter activity;
(c) a DNA containing a base sequence derived from the base sequence shown under SEQ ID NO:12 by deletion, substitution or addition of at least one base and having promoter activity.
(d) a DNA derived from'a yeast belonging to genus Candida, which hybridizes with base sequence of SEQ ID NO:12 under stringent condition and has a promoter activity.

**17.** A DNA
which comprises the TEF1 gene promoter according to Claim 16 and a structural gene joined to the promoter sequence downstream therefrom.

**18.** A gene expression unit
which comprises the DNA according to Claim 17 and a terminator.

**19.** A plasmid
which contains the gene expression unit according to Claim 18.

**20.** The plasmid according to Claim 19,
which is pUTA-TEF1-ORF2S.

**21.** A transformed cell as resulting from transformation of the DNA according to Claim 2, 7, 12 or 17.

**22.** A transformed cell as resulting from transformation of the plasmid according to Claim 4, 5, 9, 10, 14, 15, 19 or 20 into a host cell.

**23.** The transformed cell according to Claim 21 or 22,
wherein the host cell is *Candida maltosa*.

**24.** The transformed cell according to any of Claims 21 to 23,
wherein the structural gene is an *Aeromonas caviae*-derived gene encoding a enzyme involved in the synthesis of the copolymeric polyester resulting from copolymerization of 3-hydroxybutyric acid and 3-hydroxyhexanoic acid.

**25.** A method of producing the copolymeric polyester resulting from copolymerization of 3-hydroxybutyric acid and 3-hydroxyhexanoic acid
which comprises culturing the transformed cell according to Claim 24.

Fig. 1

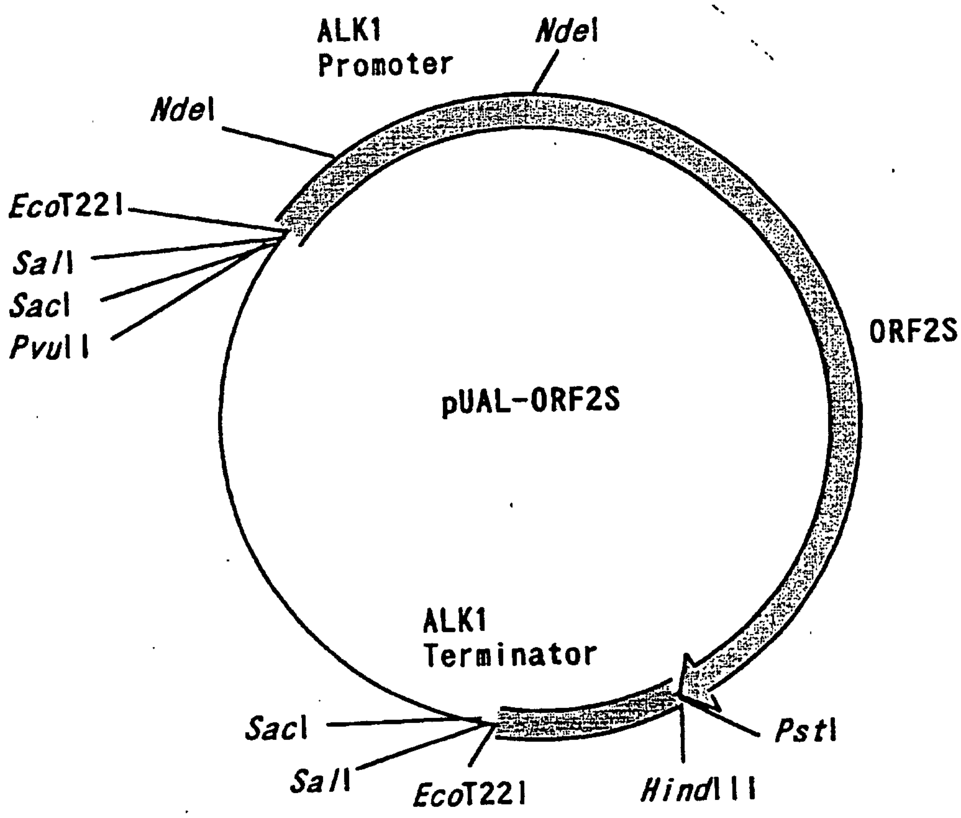
ALK1
Promoter
NdeI
NdeI
EcoT22I
SalI
SacI
PvuII
ORF2S
pUAL-ORF2S
ALK1
Terminator
SacI
SalI
EcoT22I
HindIII
PstI

Fig. 2

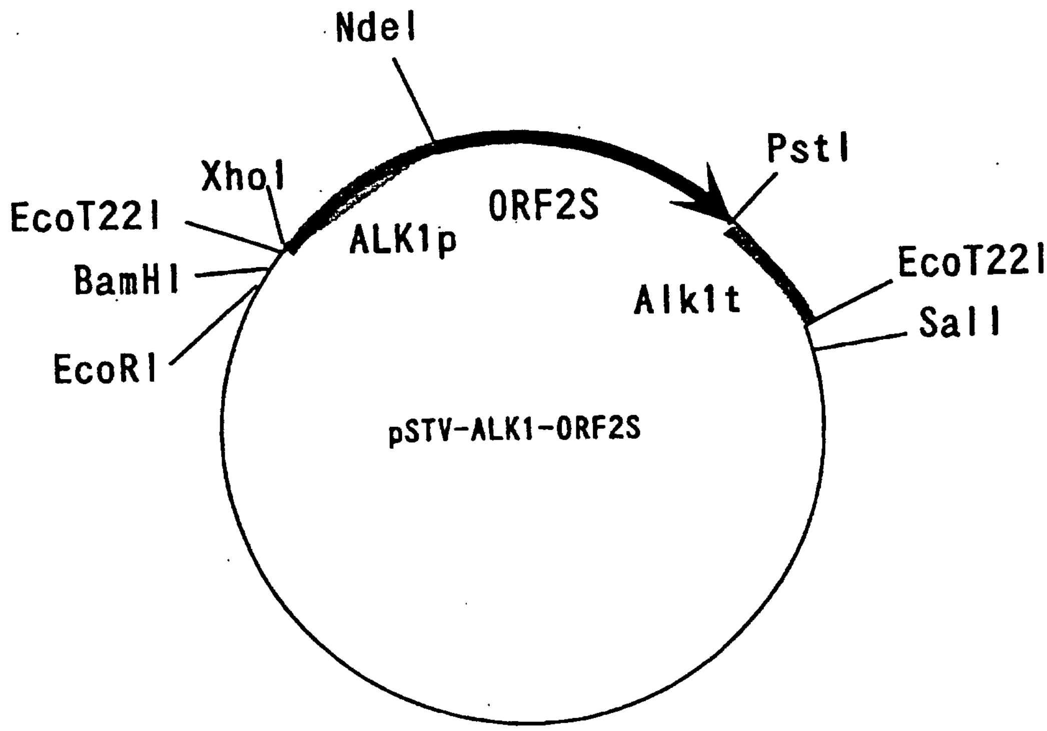
NdeI
XhoI
EcoT22I
BamHI
EcoRI
ALK1p
ORF2S
PstI
EcoT22I
Alk1t
SalI
pSTV-ALK1-ORF2S

Fig. 3

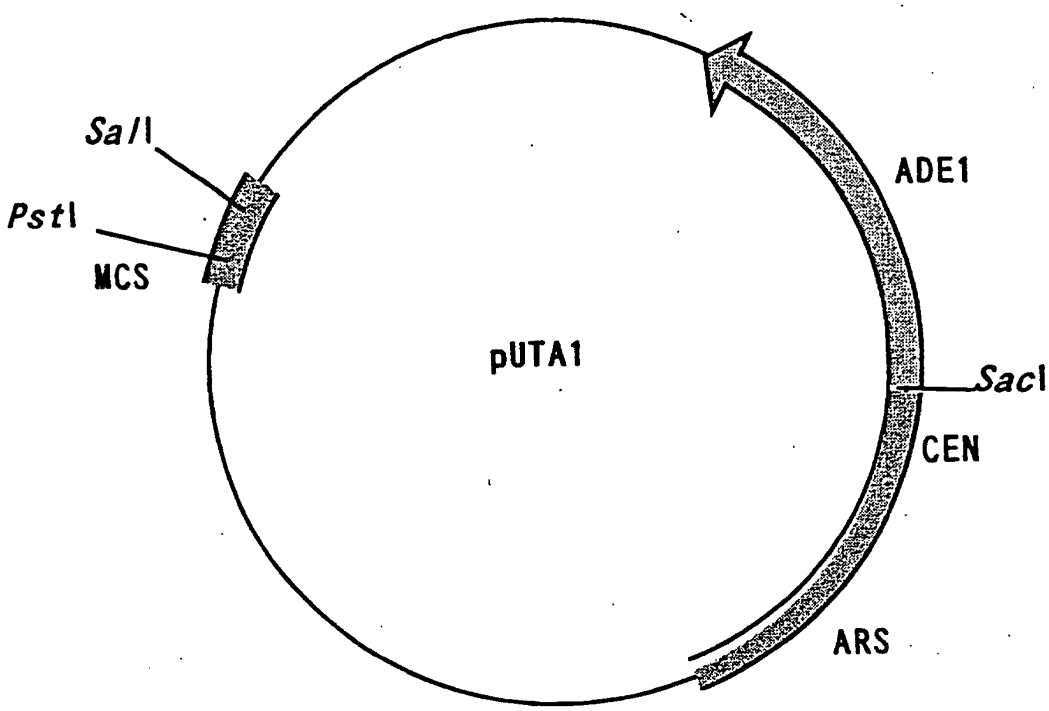
SalI
PstI
MCS
ADE1
pUTA1
SacI
CEN
ARS

Fig. 4

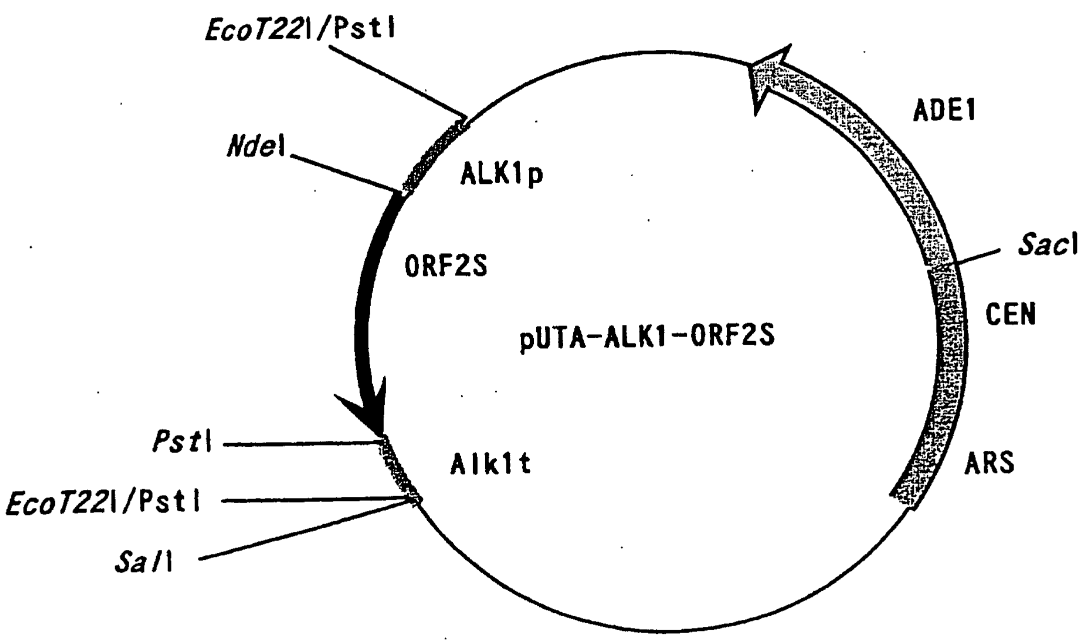
EcoT22I/PstI
NdeI
ALK1p
ORF2S
pUTA-ALK1-ORF2S
ADE1
SacI
CEN
PstI
Alk1t
ARS
EcoT22I/PstI
SalI

Fig. 5

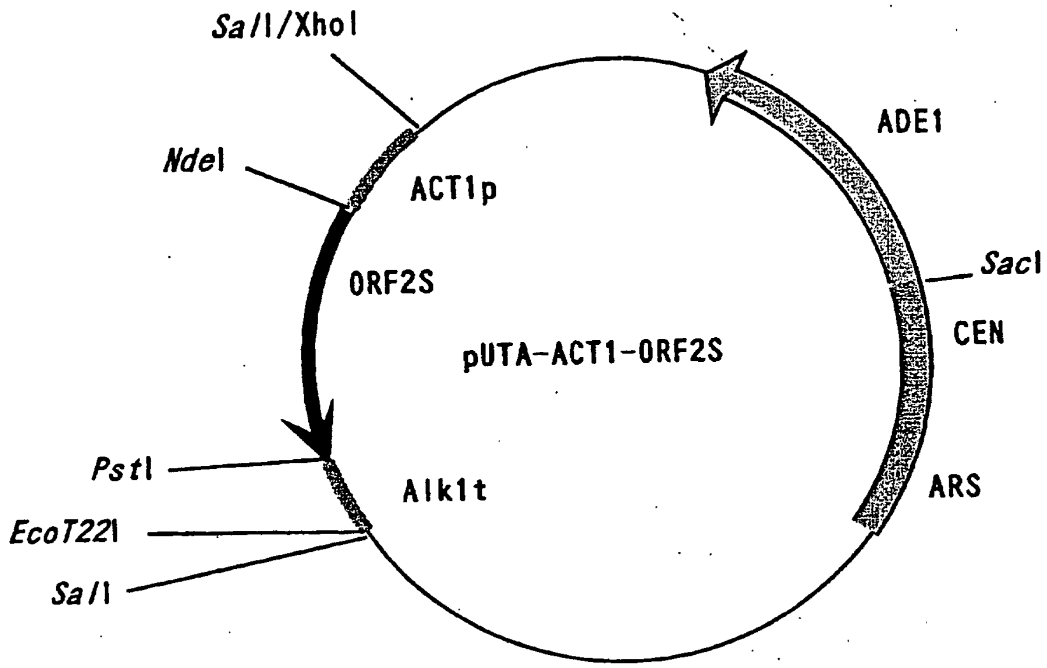
SalI/XhoI
NdeI
ACT1p
ORF2S
pUTA-ACT1-ORF2S
PstI
EcoT22I
SalI
Alk1t
ADE1
SacI
CEN
ARS

Fig. 6

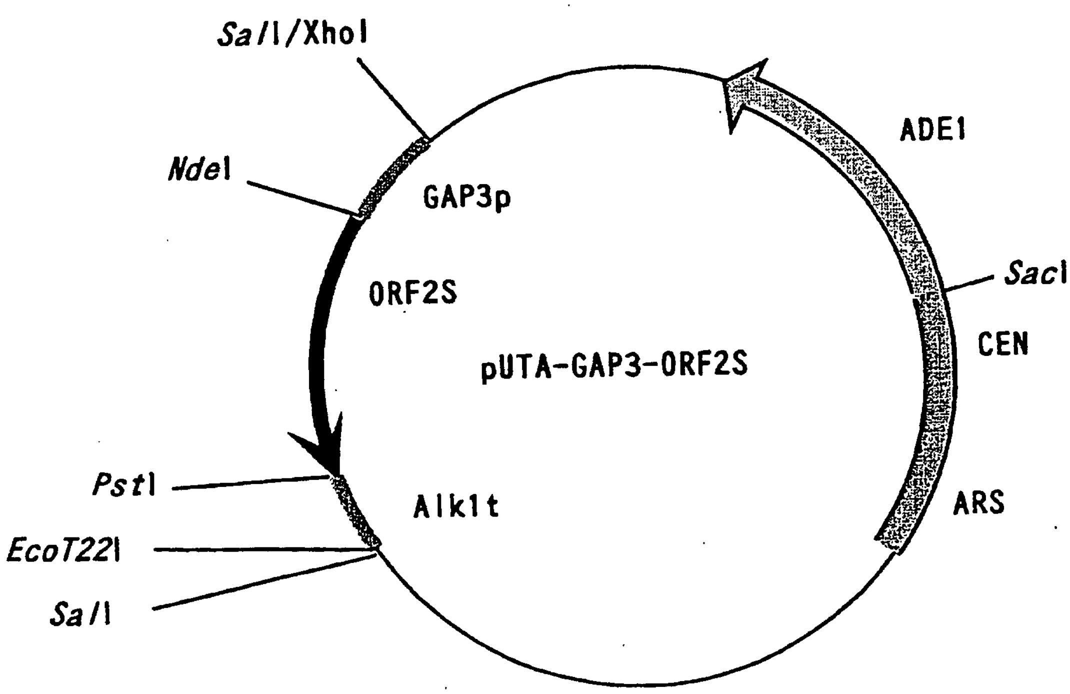
SalI/XhoI
NdeI
GAP3p
ORF2S
pUTA-GAP3-ORF2S
PstI
EcoT22I
SalI
Alk1t
ADE1
SacI
CEN
ARS

Fig. 7

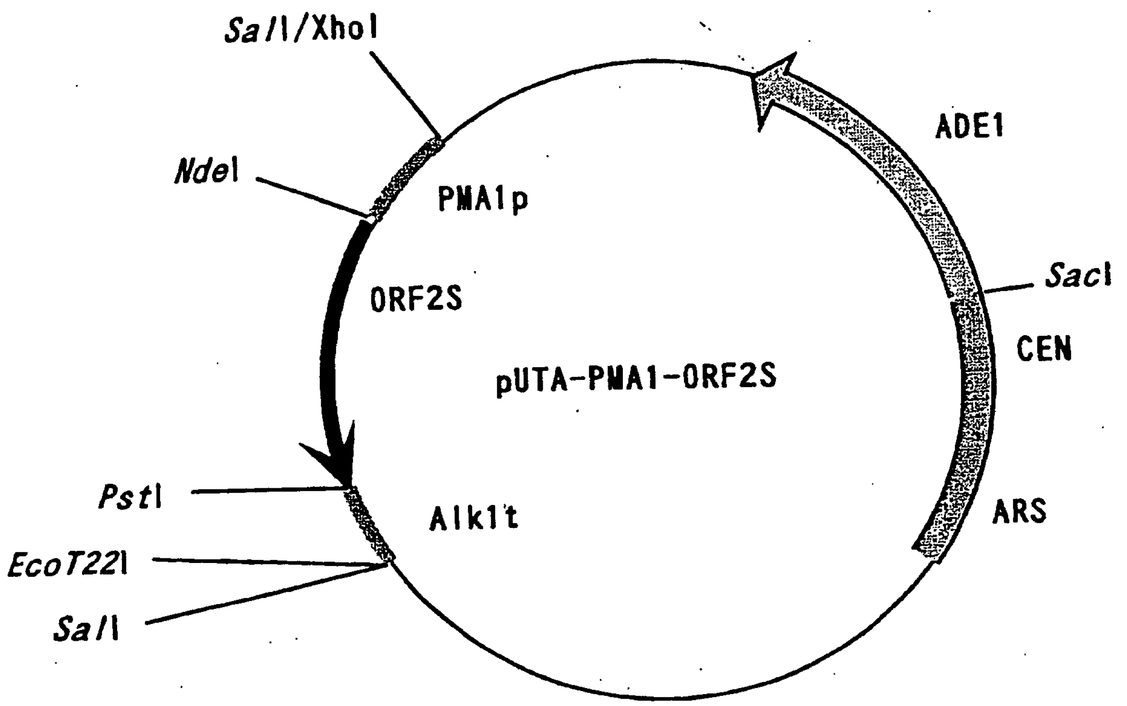
SalI/XhoI
NdeI
PMA1p
ORF2S
pUTA-PMA1-ORF2S
PstI
Alk1t
EcoT22I
SalI
ADE1
SacI
CEN
ARS

Fig. 8

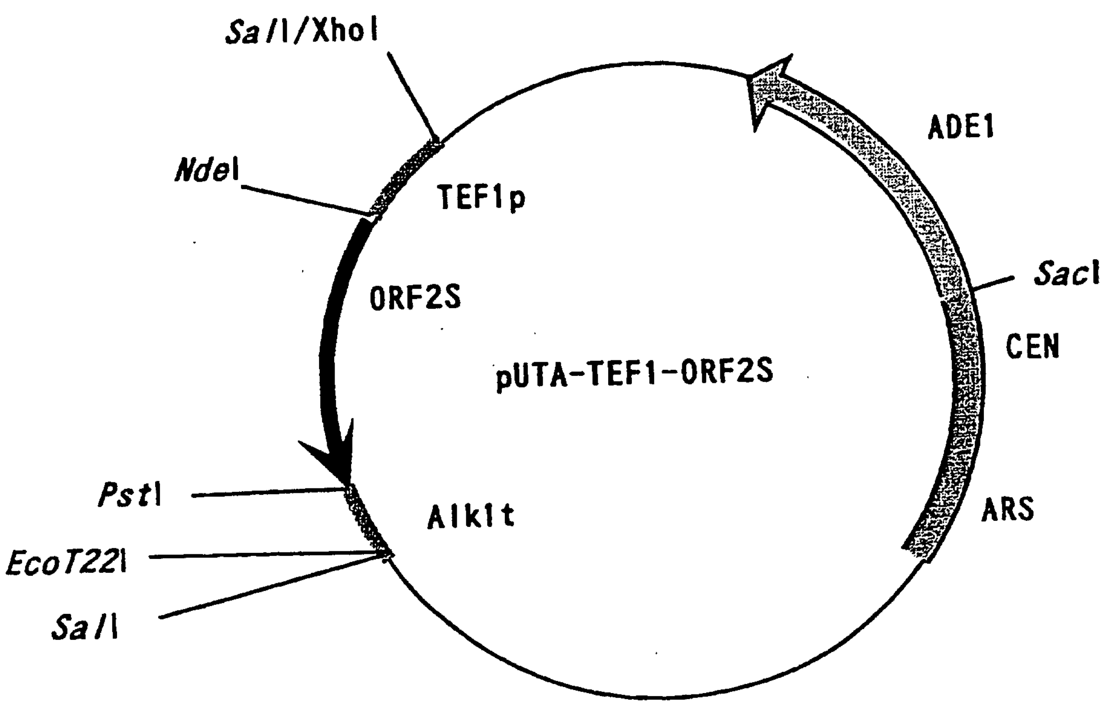
SalI/XhoI
NdeI
TEF1p
ORF2S
pUTA-TEF1-ORF2S
PstI
Alk1t
EcoT22I
SalI
ADE1
SacI
CEN
ARS

## INTERNATIONAL SEARCH REPORT

International application No.
PCT/JP03/04426

A. CLASSIFICATION OF SUBJECT MATTER
Int.Cl[7] C12N15/11, 1/19, C12P7/62//(C12N1/19, C12R1:72)

According to International Patent Classification (IPC) or to both national classification and IPC

B. FIELDS SEARCHED

Minimum documentation searched (classification system followed by classification symbols)
Int.Cl[7] C12N15/00-15/90

Documentation searched other than minimum documentation to the extent that such documents are included in the fields searched

Electronic data base consulted during the international search (name of data base and, where practicable, search terms used)
GENBANK/EMBL/DDBJ/GENESEQ, BIOSIS/MEDLINE/WPIDS(STN), JSTPLUS(JOIS)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | LOSBERGER C. et al., Sequence of the Candida albicans gene encoding actin. Nucleic Acids Res., 1989, Nov 25, 17(22), page 9488 | 1-4,21-23<br>5,24,25<br>6-20 |
| X<br>Y<br>A | GIL-NAVARRO I. et al., The glycolytic enzyme glyceraldehyde-3-phosphate dehydrogenase of Candida albicans is a surface antigen. J.Bacteriol., 1997 August, 179(16), p.4992-9 | 6-9,21-23<br>10,24,25<br>1-5,11-20 |
| X<br>Y<br>A | MONK B.C. et al., Cloning and characterization of the plasma membrane H(+)-ATPase from Candida albicans. J.Bacteriol., 1991 November, 173(21), p.6826-36 | 11-14,21-23<br>15,24,25<br>1-10,16-20 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 June, 2003 (03.06.03) | 17 June, 2003 (17.06.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/04426

| C (Continuation). DOCUMENTS CONSIDERED TO BE RELEVANT | | |
|---|---|---|
| Category* | Citation of document, with indication, where appropriate, of the relevant passages | Relevant to claim No. |
| X<br>Y<br>A | SUNDSTORM P. et al., Sequence analysis and expression of the two genes for elongation factor 1 alpha from the dimorphic yeast Candida albicans. J.Bacteriol, 1990 April, 172(4), p.2036-45 | 16-19,21-23<br>20,24,25<br>1-15 |
| Y<br><br>A | FUKUI T. et al., Co-expression of polyhydroxyalkanoate synthase and (R)-enoyl-CoA hydratase genes of Aeromonas caviae establishes copolyester biosynthesis pathway in Escherichia coli. FEMS Microbiol Lett, 01 January, 1999 (01.01.99), 170(1), pages 69 to 75 | 5,10,15,20, 24,25<br>1-4,6-9, 11-14,16-19, 21-23 |
| A | MUTOH E. et al., A gene coding for a ribosomal protein L41 in cycloheximide-resistant ribosomes has a promoter which is upregulated under the growth-inhibitory conditions in yeast, Candida maltosa. Biochem.Biophys.Res.Commun., 19 May, 1999 (19.05.99), 258(3), p.611-5 | 1-25 |
| A | OHKUMA M. et al., Evidence that the expression of the gene for NADPH-cytochrome P-450 reductase is n-alkane-inducible in Candida maltosa. Biosci.Biotechnol.Biochem., 1995 July, 59(7), p.1328-30 | 1-25 |

Form PCT/ISA/210 (continuation of second sheet) (July 1998)

## INTERNATIONAL SEARCH REPORT

International application No.
PCT/JP03/04426

**Box I Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

The Candida Maltose-origin promoters not restricted to carbon sources as set forth in claims 1 to 5 and 11 to 20 and the Candida Maltose-origin promoters as set forth in claims 6 to 10 are common to each other exclusively in being a Candida Maltose-origin promoter. However, Candida Maltose-origin promoters had been publicly known (Curr Genet, 1994, 25, p.412-417) and thus these groups of inventions are not considered as a group of inventions so linked as to form a single general inventive concept. Thus, there is no special technical matter common to all claims.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1998)

SEQUENCE LISTING

<110> KANEKA CORPORATION

<120> A new promoter

<130> T747.SBP-7

<150> JP 2002-105240

<151> 2002-4-18

<160> 27

<210> 1

<211> 29

<212> DNA

<213> Artificial Sequence

<220>

<223> PCR Primer for Sc-ACT1 5'

<400> 1

ccggaattca tggattctgg tatgttcta 29

<210> 2

<211> 29

<212> DNA

<213> Artificial Sequence

<220>

<223> PCR Primer for Sc-ACT1 3'

<400> 2

ccggaattca agacagcacg aggagcgtc 29

<210> 3

<211> 30

<212> DNA

<213> Artificial Sequence

<220>

<223> PCR Primer for Sc-GAP3 5'

<400> 3

atgatcagaa ttgctattaa cggtttcggt 30

<210> 4

<211> 29

<212> DNA

<213> Artificial Sequence

<220>

<223> PCR Primer for Sc-GAP3 3'

<400> 4
ttaagccttg gcaacatatt cgatcaagt 29

<210> 5
<211> 30
<212> DNA
<213> Artificial Sequence

<220>
<223> PCR Primer for Sc-PMA1 5'

<400> 5
atgactgata catcatcctc ttcatcatcc 30

<210> 6
<211> 30
<212> DNA
<213> Artificial Sequence

<220>
<223> PCR Primer for Sc-PMA1 3'

<400> 6
ttaggtttcc ttttcgtgtt gagtagagac 30

<210> 7

<211> 30

<212> DNA

<213> Artificial Sequence

<220>

<223> PCR Primer for Sc-TEF1 5'

<400> 7

atgggtaaag agaagtctca cattaacgtt 30

<210> 8

<211> 30

<212> DNA

<213> Artificial Sequence

<220>

<223> PCR Primer for Sc-TEF1 3'

<400> 8

ttatttctta gcagcctttt gagcagcctt 30

<210> 9

<211> 1300

<212> DNA

<213> Candida maltosa

<220>

<223> Cm-ACT1 Promoter

<400> 9

```
gatctcggct gtgaatcgca atttgccatg acacctctcg ctatttccga attacataag 60
accatcagtg aaagattgga gagaaaaaaa tgtgaacaga atagtcggag tttatattaa 120
attcatcgtc caaaaaaacc tgaatatcat tggctctgcg gtttattaaa aagtgtaccg 180
ccgacttatc cgaacaatta aacgtaacat gactgcaaaa aaaaaccatg aagaaaacat 240
ttacaaaata tttgtaatat cgtcgaagat aataaaccat accagacgga aagttggatg 300
aaattgttgc aaaaataatt atgctactcg ttctcacgtt taaatataca cgtagatcaa 360
accagcaaac ttctataaat tgccatcgat ccaccaaaca tcgtcaaaaa caattttgta 420
actttattgc ctctcatacg tttaaatttc aaaatcaata aatattaatc aaccgtgtaa 480
caaaaaaaaa aaactgagat agtgcacagc ccaaaaaggt ttagtgattg cctccaaaca 540
tacataatag gttatttttt tcgttgaacg catttcttgc tcgctcaccg aatttctgcc 600
aacgaaacaa attttatata tttcattttt ttctctttca tgtaattttt tctttctttc 660
tgctttttct tttttttttc ttttccttcc caatcccccc ctacattaat atattaatta 720
tcatttaaaa tggacggtgg tatgtttaaa ctatttcatt gacttgattg atcgattgat 780
tgattggttg atttcttcaa agcacggact ttttttcttt ctccattatt tgatttttag 840
attttgggtg atttttttgt ttttttttggg gagtgactga tctaatctca attcaggatt 900
atgggacgaa gaagaatcga gagatggaat ctagatatat caatttcaat ttttattgtt 960
ttgatctcga gagtatttat ggaaagattt gattgaacaa cttttttttt cattggcctg 1020
gatcaattcc gtccataaaa gaaagagagc tttacactga tcgattcatt catatttttt 1080
acactggagt ttcttcaaaa atcattggat tctacccaat ggcaatccta catcttaaaa 1140
atcatcattt attacgagtt tattggataa tggtcacttt aaattcttgg tatcttgaat 1200
ttgttttttt tttttttttt tttcagaaat ttgtaccccc ttttaaaaat gttcagaatt 1260
tttttttttt tttcgtcaaa cccacacaca cacatttttc                       1300
```

<210> 10

<211> 3000

<212> DNA

<213> Candida maltosa

<220>

<223> Cm-GAP3 Promoter

<400> 10

ctgcaggtgg gatcttcaga ctgtttaata tttcttcaat atgatgggtc ccataattag 60

aaaccccaat actcttaaca atccccttgg acacggcttc ttccatgact ttccaacttt 120

ccaatcgttt ttgtttccca ggtaatggtg aatggatcaa taacaaatcg atatatttca 180

aatcaccaat ttgatccaac atagtagtga tcgcatgtct tgtatttgtt gtacccaatt 240

gactattcca tagtttggtg gtatagaaaa actctgaacg agggatatct ggattatcgt 300

gaaggaattt cgttatccct tctgccactt cttcttcgtt gccgtacaaa actgcggtat 360

caaaatgtcg atatccgact ttacaggctt cgtagacaat gctggccgtt ttatttcttg 420

gaatgtcgta acagcctaaa ccgattgaag ggatgctata tccggaattg agtttgatta 480

atcgaaatga catgattgtg ttgagtatat tgaaagcaat aattaatata aaaaaaagag 540

gaacgaaaaa aaagagagat gttgaagtgg ttgggttatg taagtacgta tttattcact 600

gattattaat tgctatctta ataatatttt tttccctccc atttttactt tttttggata 660

tcttgtttga aatgtggggg caaaaaaaaa aaaaatttac atagccctat tccataaaat 720

ataaatcttt tatgtatatt tgcaacatcg acacaatttg atatttccaa atactccagg 780

tttttttttc tttttcattc acagtctcgg gattaagtgt gaaacccggg ggaaatcgaa 840

attttttttt ttcagcattg tttatacaca atttcagttt gtccgaatac acccgcacgt 900

gattccccca aacaggcaaa aaaaaaaaaa aatgaatata tagtgagtac gtgtcccgcg 960

gctcaggaac ctcttttttt tttagaggtg gtatgatgtg aagtattttt tttttttcct 1020

ttttcctttt ccttttttcat tcacaccacc accatataga atttacttac gtcaggttat 1080
attctagaca acctttgtgg tttttttttt taaagggaat ttgagccact atgtccatag 1140
aaaacttttt actgtaacga aaatctatag tctgagataa aggggaaaat ggtaaccacg 1200
tatttttta ttttttttg gattcctata accccgatat ttatgttcgg aattgtagat 1260
atatagatat tccagattac ttggctgtaa tgtaggctat ggaaatgata ctactcatca 1320
atataaaccc attgacagta taagatagat aattatactg tggtggtacc atataaaatt 1380
aatatgttga tcaggtgctt ttggcaacac cacgagcttt gcgcaagttt tttttttttg 1440
ttctttttg tttttgttg gttgtttgat gcaaatggat gataatgccc cgggcgcggg 1500
cgtgtgtgac gcaaatccaa tagaaaaat tcacctggtt aaacctattt tcactgacaa 1560
atcaatttat tttgccaaaa gaaaaaaaga atatataata acccttgaat gtccaattgg 1620
aattttttt ctctttctaa aatttttctt tctttctttc ttttcttctt cttttcttct 1680
ttacacaatc aattgacttt aaacctcaat taaacaacac ataactttca aacttacttt 1740
ttaacataca aaaaatggct attaaaattg gtattaacgg tttcggtaga atcggtagat 1800
tagtcttgag aattgcttta ggcagaaaag acattgaagt tgttgccgtc aacgatccat 1860
tcattgctgc tgattacgct gcttacatgt tcaaatacga ttccacccac ggtagataca 1920
aaggtgaagt caaatctgaa ggtaacgatt tagtcattga cggtaagaaa atccaagtct 1980
tccaagaaag agacccagct aacattccat ggggtaaaga aggtgttgaa tatgttattg 2040
actccactgg tgttttcacc aagattgaag gtgctcaaaa acacattgat gctggtgcca 2100
aaaaagttat catcactggt tcatctgctg atgctccaat gttcgttgtt ggtgttaacg 2160
aagacaaata caccccagac ttgaaaatca tttctaacgc ttcctgtacc actaactgtt 2220
tagctccatt agctaaagtt atcaacgata ctttcggaat tgaagaaggt ttgatgacca 2280
ctgtccactc catcactgct acccaaaaga ctgttgacgg tccttcccac aaagattgga 2340
gaggtggtag aactgcttcc ggtaacatta tcccatcttc tactggtgct gctaaagccg 2400
tcggtaaagt tatcccagaa ttaaacggta aattgactgg tatgtctttg agagttccaa 2460
ccaccgatgt ctccgttgtt gacttgactg tcagattatc taaaccaacc acttacgaag 2520
aaatctctga agctatcaag aaagctgctg atggtccatt gaacggaatc ttgggttaca 2580
ctgaagatgc tgttgtctct actgacttct tgtcttctaa ctactcttct gttttcgatg 2640
ctaaagctgg tatcttgttg tccccaactt tcgtcaaatt gatctcttgg tacgataacg 2700

aatacggtta ctctaccaga gttgtcgact tattggaaca cgttgccaaa gtttccggtt 2760

cctcttaact cagaaacaag ttttagttga cattgtgtct gttttctttt attacatagg 2820

ttgttatatc aatatatgtt tataaatacg tcttgaaaat cttgtttttt tttttgtaa 2880

attttgtaaa ttttcatctt gtgcgggaca aaggacgagt ggagaaaaaa aaaacgaaac 2940

ttttttttc ttttctccga aattgtaaac aaaaacaaca acaacacctc catgtcggaa 3000

<210> 11

<211> 3173

<212> DNA

<213> Candida maltosa

<220>

<223> Cm-PMA1 Promoter

<400> 11

tctagaattt atattggttt ctttcttttt ttttagatcg tttattaatt aattagttaa 60

ttaattactt cataacatgt aaattagatt taaccaaaaa aaagaaaagt taaagataat 120

ggctaagtag atgttaaagc caggttcaat tgtttataat actcatcatc atcaatcaat 180

taaattgcaa aaacaaacaa acccgttgaa aaaaggaaag aaaaaaaaaa cacggctggt 240

aataaatctt tcatgtactg gtcaattaat taaccgacgt aataagagat ccttggataa 300

atagtaagaa tatccagcaa tttacgtacg taaatgaaac acaaatgaat gaatgctgaa 360

ctttcatgac ttaattgagt agtttagttg gttggtatat gcgaaattta tttattccga 420

taattattat caataggttg tagcgggaaa tttaaaacca aacaggagat tagaagcggc 480

agaatgaaat aaaaaaaaac atggccggcg aaaaaaaaaa ggaaaaaaag gaaggaaaaa 540

aaaacgaaaa agggtcgggt aaatctactc aacaaatatt ataataatga ttgtttattt 600

atctatggat gtttggatga attaagtcaa gtttgtgtta tttcgtatga aagagacata 660

gttagagata gagatagata gacaaataga tttgagagat gaggtggttc agttacatta 720

catttttttt tcttaaaaac taaaaatatt tcttatgtaa ctttccagtt tattatatta 780
ataccaagaa agttatatgt aatatcagtt gatattcaac aattgctgtt acaattgtca 840
actctcaact tctaccttcc catttgaata tctctcttcc agtcatatga gttgtattca 900
aaattttttt tatttccgtt cggcataatt aattttgtgt cgtgggaata tgcacaattt 960
ataaaacaaa agcaaaatct aaattgaggg aatttctgca gaagagtcaa aaaaaacata 1020
aagtcgtgtc tcggaactca aaaataacat tttccatact aagattaaac gataacattt 1080
aaaaaaatcc acaaatttga ttggtcggaa taaaaaataa aaatatccct caccctaaag 1140
aaagaaaatt tttttattta gttgagaaaa ccgaataatt ttgtcctatg aggtaattaa 1200
atatttccat tttgtgttat tgtttattat tttcctaaac cactttatca aaaaaagaaa 1260
aagaaatttt tcttcttttt tggacaaaat taaaaatttt ttacaacctc ttctaaaaaa 1320
gaaaaacaac aacaacagaa aaacgacctc caaaaaaatc ttacaaccaa aaatttaaat 1380
ttttaatttt ccaaaggtaa tataaaaagg ataataaatt cccttgatta gatttttttt 1440
tttaacgaat tctttattca tttttccttt ttcccttttt ttttttttcc ttttttttaga 1500
tagtcaatcg aagttttact tttattaact ttttttccta cccactaatt cttactttct 1560
tttttttttc attcaaaaat tttttaatag tattttaaaa aatataccat ctcacacccc 1620
caaaaaagaa aaataaaaag gaattcattt ttaataccct aatttttttaa tattagaatt 1680
atagagagag aaaaagagac agaaaacaaa aacttatcat gagtgctact gatcctacta 1740
atgaaaagat caataaagac atctccgatg atgaagatga agatattgat caattggttt 1800
tagatttaca atctaatcca ggtgctttag atgacgaaga agaagaagaa gatcaagctt 1860
cttttaaagc cgtccctgaa gaattattac aaactgaccc aagaactggt ttatctgatg 1920
atgaagttca aaaaagaaga aaaaagtatg gtttgaatca aatggctgaa gaacaagaaa 1980
atttagtcat gaaattcgtc atgtttttcg ttggtccaat tcaattcgtt atggaagccg 2040
ctgctgtttt agctgctggt ttggaagatt gggtcgattt tggtgttatc tgtgctttat 2100
tggtattgaa tgcttttgtt ggtttcatcc aagaatacca agctggttct attgtcgatg 2160
aattaaaaaa gactttagct aacgttgctt tagttgttag aaatggtcaa ttggttgaaa 2220
ttccagccaa tgaagttgtt ccaggtgata tcttgcaatt ggaagatggt accgttatcc 2280
caactgatgg tagaattgtt tctgaagatt gtttattaca agttgatcaa tctgctatta 2340
ctggtgaatg tttagctgtt gacaaaagat ctggtgactc ttgttactct tcttccactg 2400

ttaaaactgg tgaagctttt atggttgtta ccgctactgg tgacaacact tttgttggta 2460
gagctgctgc tttagtcaac aaagcttccg ctggtactgg tcatttcact gaagttttga 2520
acggtattgg tactacattg ttggttttcg tcattgttac tttgttggtt gtctgggttg 2580
cttgtttcta cagaactgtt agaattgttc caatcttgag atacactttg gctatcacca 2640
ttattggtgt tccagtcggt ttaccagctg tcgttaccac taccatggct gtcggtgctg 2700
cttacttggc taaaaaacaa gctattgtcc aaaaattgtc tgctattgaa tctttggctg 2760
gtgtcgaaat tttatgttct gataaaactg gtactttgac caagaataaa ttgtctttac 2820
atgaaccata cactgttgaa ggtgttgaac cagatgactt gatgttgact gcttgtttgg 2880
ctgcttccag aaagaagaag ggtttggatg ctattgataa agctttcttg aaatctttga 2940
ttaactaccc aagagctaaa gctgctttac caaaatacaa agttattgaa ttccaacctt 3000
ttgatcctgt ctccaaaaaa gttactgcca ttgttgaatc accagaaggt gaaagaatta 3060
tttgtgttaa gggtgctcca ttattcgtct tgaagactgt tgaagatgcc catccaatcc 3120
cagaagatat ccatgaaaac tatcaaaaca ctgttgccga atttgcttct aga 3173

<210> 12

<211> 1675

<212> DNA

<213> Candida maltosa

<220>

<223> Cm-TEF1 Promoter

<400> 12

ctgcagcagc ttctactgct gccgctccaa cattaggtgc tgaatatact agcggtactg 60
gtaaattagt tggtgtggtt acattgactg atattttggg attatttgcc acatcaaaag 120
gtagaagaac tgatccacaa gctgcaagaa accaaagaag aagaagttcc acttccacta 180
cgagatcatc tgttgatagt gcattaaacg ctgaaggtgt gattaatcct tctgccacca 240

ccaccaccga tgccattcct ggtaataaca attctagtcg tagagaaagt gttgatgctt 300
caagtgatgt tttcagaaaa tcatttacta aaccccaaga aaatgtattt tccaaagagt 360
aagggttcct tctttcataa caaaaaaaga aaaacaatca ccgatttatt tatttatttg 420
caatgctatt tataatatat tttgtagata aaaacaaatg aaaaatcttg ttagctatgt 480
atactactac atatatacta caataaaaac acaccaaaat gaaacgtgtt ttgcacaatt 540
tcgcacgact cagaggcatc gcatttctgt ccttttgta cgtcattgta attttttta 600
tgttattttt tttacagcaa gcaatccaaa aaaacaaaaa aaaaatgaga gagaaaaaaa 660
tgaggggtt gatttaaaaa gatggtcaaa aatattcgtg acatattaca taatcgatga 720
gtttgatatg gaacgaatat tgatggtttt ggtctgaatt gatatggtgt aagtatttgt 780
tggtgataat tttatcaaca taaactcaat tccgctcaat tgtacaaaat tgaccttctt 840
tcgcctttg ttcaatgcca tttttccaa taattttttt tttcaaattt tgccatccag 900
cacaaagaaa aaaaaaattt acatgtccga caactcaccg gtgtttctga caacaattga 960
caacaccagt ctgtagaccc aattggtaag tcaatgataa ctactacatc tacctagttg 1020
ttatctttta acttaaaatt agcaaagaaa taataatggt tatcattgaa gatggtttca 1080
caaaattaaa cgaatacgtg tacgttttac caaaaagatt ttttttttc tctttagttt 1140
tttttcgtt gttcttccca tcactgaaaa atttttctcc ctctatataa atcaatccca 1200
tcaacgaaaa ttttttttct tcctttttga attttttttt tctccttttt ttttctcctt 1260
ttttttctc cttttctttc ttcatctaac ttatatttaa tcaatcatgg gtaaagaaaa 1320
aactcacgtt aacgtcgttg ttattggtca cgtcgattct ggtaaatcta ctaccaccgg 1380
tcacttgatc tacaagtgtg gtggtattga caaaagaacc attgaaaaat tcgaaaaaga 1440
agctgctgaa ttaggtaaag gttctttcaa atacgcttgg gtcttggata aattgaaggc 1500
tgaaagagaa agaggtatca ccattgatat cgctttgtgg aaattcgaaa ctccaaaata 1560
ccacgttacc gttattgatg ctccaggtca cagagatttc atcaaaaata tgattactgg 1620
tacttctcaa gctgattgtg ctattttgat tattgctggt ggtactggtg aattc 1675

<210> 13

<211> 1794

<212> DNA

<213> Artificial Sequence

<220>

<223> ORF2S

<400> 13

atg tct caa cca tct tat ggt cca ttg ttc gaa gct ttg gct cat tac 48

aat gat aaa ttg ttg gct atg gct aaa gct caa acc gaa aga act gct 96

caa gcc ttg ttg caa act aac ttg gat gat ttg ggt caa gtt ttg gaa 144

caa ggt tct caa caa cca tgg caa ttg att caa gct caa atg aat tgg 192

tgg caa gat caa tta aaa ttg atg caa cac act ttg tta aaa tct gct 240

ggt caa cca tct gaa cca gtt att act cca gaa aga tct gat aga aga 288

ttt aaa gct gaa gct tgg tct gaa caa cca att tat gat tac tta aaa 336

caa tcc tat ttg tta act gct aga cat ttg ttg gct tct gtt gat gct 384

ttg gaa ggt gtc cca caa aaa tct aga gaa aga ttg aga ttc ttt act 432

aga caa tac gtc aac gct atg gct cca tct aat ttc ttg gct act aac 480

cca gaa ttg tta aaa ttg act ttg gaa tcc gat ggt caa aat ttg gtt 528

aga ggt ttg gct tta ttg gct gaa gat ttg gaa aga tct gct gat caa 576

tta aac att aga ttg act gat gaa tcc gct ttt gaa tta ggt aga gat 624

ttg gct ttg act cca ggt aga gtt gtt caa aga act gaa tta tat gaa 672

tta att caa tac tct cca act act gaa acc gtt ggt aaa acc cca gtt 720

ttg atc gtt cca cca ttc att aat aaa tat tac att atg gat atg aga 768

cca caa aac tcc ttg gtc gct tgg ttg gtc gct caa ggt caa acc gtt 816

ttc atg att tcc tgg aga aac cca ggt gtt gct caa gct caa att gat 864

tta gat gat tat gtt gtt gat ggt gtc att gct gct ttg gat ggt gtt 912

gaa gcc gct act ggt gaa aga gaa gtt cac ggt att ggt tac tgt att 960

ggt ggt acc gct ttg tct tta gct atg ggt tgg ttg gcc gcc aga aga 1008

caa aaa caa aga gtt aga act gct act ttg ttt act act ttg ttg gat 1056

ttc tcc caa cca ggt gaa ttg ggt att ttt att cat gaa cca att atc 1104

gcc gcc tta gaa gcc caa aat gaa gct aaa ggt att atg gat ggt aga 1152

caa ttg gcc gtc tcc ttc tct ttg ttg aga gaa aac tct tta tat tgg 1200

aat tac tat att gat tct tac tta aaa ggt caa tct cca gtt gct ttt 1248

gat ttg ttg cac tgg aac tct gat tct act aat gtt gcc ggt aaa act 1296

cat aac tct ttg ttg aga aga tta tat ttg gaa aat caa ttg gtt aaa 1344

ggt gaa tta aaa att aga aac act aga att gat tta ggt aaa gtt aaa 1392

act cca gtt ttg ttg gtt tct gcc gtt gat gat cac att gct tta tgg 1440

caa ggt acc tgg caa ggt atg aaa ttg ttc ggt ggt gaa caa aga ttt 1488

tta ttg gcc gaa tcc ggt cat att gct ggt att att aat cca cca gct 1536

gct aac aaa tac ggt ttc tgg cac aat ggt gct gaa gct gaa tct cca 1584

gaa tct tgg ttg gct ggt gcc acc cat caa ggt ggt tcc tgg tgg cca 1632

gaa atg atg ggt ttt att caa aac aga gat gaa ggt tct gaa cca gtc 1680

cca gcc aga gtc cca gaa gaa ggt ttg gct cca gct cca ggt cac tat 1728

gtc aaa gtt aga tta aac cca gtt ttc gct tgt cca acc gaa gaa gat 1776

gct gct tct aaa ttg taa 1794

<210> 14

<211> 218

<212> DNA

<213> Candida maltosa

<220>

<223> terminator ALK1t

<400> 14

Atagatggat ttttcttttt tatgtgtatt tccggttaat aaatgtttaa attttttttt 60
taataaaaat atttgtagtt atttatatgc aaaaaaaaaa aatattcaaa gcaatcttcc 120
tttctttctt tatctttccc ccatgctaag gtctaaaaca ccacaactta aaacccaact 180
taaccgtata atactaagat caatctccaa agatgcat 218

<210> 15
<211> 1017
<212> DNA
<213> Candida maltosa

<220>
<223> promoter ALK1p

<400> 15
atgcatgaac aggatttaat cccaagaaaa aagtctattt tctattttca caaggaaact 60
ggaaaaacct ttttgtgttt tgaagtagct ccgtaataac ctgtaaaaaa ataaattttg 120
aagatttgac ttgctgatga aaatgctatc agtgtagctc tagacttgat actagactat 180
gatggcaaca catggtggtc aacgtgcaag acatcaccca atgagaagac tgctaaccag 240
aaaaaaaagg ggacaaaaga aaaactcgag agaaaaagtc aaattggtgt aaaattggct 300
atttttggta ctttcctaat ggggaaatta attgtttaaa attccagttt ttccagagtt 360
aagatttcga ccaattattt ttaatccata tgatcttcat cattatcaac ttgtgaaaaa 420
taataatcga ggtacgttta atacgagata ttagtctacg gctatgaatg ttggatatac 480
ttcattgacg atcagaagct tgattggtta ttcaggtgca tgtgtggata taaacccaac 540
aaattatcta gcaactgtgc cttccccaca ttggtcaaag aaaccctaaa gcaaattaaa 600
atctggataa ataaatcatt catttcacat tttccggtta gtataaggtt ttttaaattt 660
ttttttacag tttagccctt tcaattacca aatacggtaa caatgtgctt tgtaacatgc 720
aggggatttt ctccgttgct gttttctcca catgctttta atgtgtaata aattaaaaaa 780

attacaaaga aaaaccggca tataagcatc ggagtttaca ttgttaacta actgcaaaat 840
ggcgatgttt caaatcaaca aaatttaaaa aaaccccaaa aaaaaagtat catataaatt 900
aaactcaaaa tccttttgat tgcataaaat ttttaaatct cttctttttt ttcttttttta 960
ctttcttatc tattctattc tttttttata tatctaattc atttataaca tctggtc 1017

<210> 16
<211> 46
<212> DNA
<213> Artificial Sequence

<220>
<223> PCR Primer for ALK1p 5'

<400> 16
tttttcagct ggagctcgtc gacatgcatg aacaggattt aatccc 46

<210> 17
<211> 39
<212> DNA
<213> Artificial Sequence

<220>
<223> PCR Primer for ALK1p 3'

<400> 17
ccggaattcc atatgcagat gttataaatg aattagata 39

〈210〉 18

〈211〉 32

〈212〉 DNA

〈213〉 Artificial Sequence

〈220〉

〈223〉 PCR Primer for ALK1t 5'

〈400〉 18

cggaagctta tagatggatt tttcttttt at 32

〈210〉 19

〈211〉 45

〈212〉 DNA

〈213〉 Artificial Sequence

〈220〉

〈223〉 PCR Primer for ALK1t 3'

〈400〉 19

tttttgatatc gagctcgtcg acatgcatct ttggagattg atctt 45

<210> 20

<211> 47

<212> DNA

<213> Artificial Sequence

<220>

<223> PCR Primer for Cm-ACT1p 5'

<400> 20

cgcggatccg aattcgtcga catgcatgga tctcggctgt gaatcgc 47

<210> 21

<211> 35

<212> DNA

<213> Artificial Sequence

<220>

<223> PCR Primer for Cm-ACT1p 3'

<400> 21

gcgggatccc atatgtatcc aataaactcg taata 35

<210> 22

<211> 35

<212> DNA

<213> Artificial Sequence

<220>

<223> PCR Primer for Cm-GAP3p 5'

<400> 22
atggctatta aaattggtat taacggtttc ggtag 35

<210> 23
<211> 35
<212> DNA
<213> Artificial Sequence

<220>
<223> PCR Primer for Cm-GAP3p 3'

<400> 23
agaagcattg gagataatct tcaagtctgg agtgt 35

<210> 24
<211> 34
<212> DNA
<213> Artificial Sequence

<220>
<223> PCR Primer for Cm-PMA1p 5'

<400> 24
gaatatctct cttccagtca ctcgagttgt attc 34

<210> 25

<211> 28

<212> DNA

<213> Artificial Sequence

<220>

<223> PCR Primer for Cm-PMA1p 3'

<400> 25

ctcatatgaa gtttttgttt tctgtctc 28

<210> 26

<211> 34

<212> DNA

<213> Artificial Sequence

<220>

<223> PCR Primer for Cm-TEF1p 5'

<400> 26

gcgggatcct cgagtaaggg ttccttcttt cata 34

<210> 27

<211> 38

<212> DNA

<213> Artificial Sequence

<220>

<223> PCR Primer for Cm-TEF1p 3'

<400> 27

ttttctttac ccatatgtga ttaaatataa gttagatg 38